# EUROPÄISCHE PATENTSCHRIFT

(11) **EP 3 098 212 B1**
(45) Veröffentlichungstag und Bekanntmachung des Hinweises auf die Patenterteilung: **04.10.2023**
(21) Anmeldenummer: 16170613.0
(22) Anmeldetag: 20.05.2016
(51) Int. Cl.: B01D 15/00, C10G 25/00, B01J 20/06, B01J 20/08, C07C 2/06, C07C 2/58, C07C 5/48, C07C 7/10, C07C 7/12, C07C 7/163, C07D 307/60, C10G 45/22, C10G 45/04, C07C 7/167

(54) **WASSERSTOFF-GESTÜTZTE ADSORPTION VON SCHWEFELVERBINDUNGEN AUS OLEFINGEMISCHEN**
ADSORPTION OF SULFUR COMPOUNDS FROM OLEFIN MIXTURES SUPPORTED BY HYDROGEN
ADSORPTION SUPPORTÉE PAR HYDROGÈNE DE PRODUITS SOUFRÉS DANS DES MÉLANGES D'OLÉFINES

(30) Priorität: 28.05.2015 EP 15169655
(43) Veröffentlichungstag der Anmeldung: 30.11.2016
(73) Patentinhaber: Evonik Operations GmbH, 45128 Essen (DE)
(72) Erfinder: Peitz, Stephan, 45739 Oer-Erkenschwick (DE); Stochniol, Guido, 45721 Haltern am See (DE); Maschmeyer, Dietrich, 45657 Recklinghausen (DE); Reeker, Helene, 44227 Dortmund (DE); Bukohl, Reiner, 45770 Marl (DE)
(74) Vertreter: Evonik Patent Association

(56) Entgegenhaltungen:
- EP-A1- 0 398 251
- EP-A1- 0 600 406
- EP-A1- 1 192 981
- EP-A1- 1 686 166
- DE-A1-102005 062 354
- F. W. Melpolder ET AL: "Composition of Naphtha from Fluid Catalytic Cracking", Industrial & Engineering Chemistry, vol. 44, no. 5, 1 May 1952 (1952-05-01), pages 1142-1146, XP055014339, ISSN: 0019-7866, DOI: 10.1021/ie50509a057

## Beschreibung

Die Erfindung betrifft ein Verfahren zur Reinigung von Kohlenwasserstoffgemischen, bei welchem ein verunreinigtes Kohlenwasserstoffgemisch enthaltend Olefine mit drei bis acht Kohlenstoffatomen durch Kontaktieren mit einem festen Sorptionsmittel auf Basis von Kupferoxid, Zinkoxid und Aluminiumoxid zumindest teilweise von schwefelhaltigen Verunreinigungen befreit wird, wobei sich das Kohlenwasserstoffgemisch während des Kontakts mit dem Sorptionsmittel ausschließlich im flüssigen Zustand befindet.

Kohlenwasserstoffe sind Verbindungen, die ausschließlich aus Kohlenstoff und Wasserstoff bestehen. Die Nomenklatur der Kohlenwasserstoffe basiert auf der Anzahl der pro Molekül des Kohlenwasserstoffes enthaltenden Kohlenstoffatome. In Kurzschreibweise wird gern das Präfix Cₙ verwendet, wobei n für die besagte Anzahl steht.

C₄-Kohlenwasserstoffe sind folglich Verbindungen, die ausschließlich aus Kohlenstoff und Wasserstoff bestehen, wobei die Anzahl der Kohlenstoffatome je Molekül vier beträgt. Wichtige Vertreter der C₄-Kohlenwasserstoffe sind die Alkene und Alkane mit vier Kohlenstoffatomen, nämlich die Butene und Butane.

Alkene (synonym: Olefine) sind Kohlenwasserstoffe, die eine C=C Doppelbindung im Molekül aufweisen. Alkane (Paraffine) sind dagegen Kohlenwasserstoffe, die nur Einfachbindungen aufweisen. Sie werden deswegen auch als gesättigt bezeichnet. Alkane und Alkene mit derselben Anzahl von Kohlenwasserstoffen kommen in Rohstoffgemischen der chemischen Industrie meist gemeinsam vor. Da Alkene aufgrund ihrer ungesättigten Doppelbindung reaktiver sind, eignen sie sich als Ausgangsstoff für chemische Reaktionen. Die weitaus reaktionsärmeren Alkane lassen sich in der Regel nur als Brennstoff einsetzen. Aufgrund der höheren Reaktivität sind Olefine wertvoller als Paraffine. Je größer der Anteil an Alkenen in einem Rohstoffgemisch gegenüber den Alkanen ist, desto teurer ist der Rohstoff.

Gemische aus C₄-Kohlenwasserstoffen sind Rohstoffe der Petro-Folgechemie. Sie stammen z.B. aus Streamcrackern (so genanntes "Crack C4"), aus katalytischen Crackern (so genanntes "FCC C4" (FCC: "fluid catalytic cracking") oder "DCC C4" (DCC "deep catalytic Cracking), aus Pyrolysen ("Pyrolyse C4"), aus MTO- bzw. MTP-Prozessen (MTO: "methanol to olefins", MTP: methanol to propylene) oder Dehydrierungen von Isobutan und n-Butan. Am verbreitesten sind C₄-Kohlenwasserstoffe aus Streamcrackern (Crack C4) und aus katalytischen Crackern (FCC C4). Es werden auch Gemische von C₄-Gemischen unterschiedlicher Herkunft gehandelt, so genannter "C₄-Schnitt". Zum Zwecke der Verwertung der einzelnen Komponenten sind die C₄-Gemische möglichst sortenrein in ihre Bestandteile zu zerlegen.

Die Aufarbeitung von C₄-Strömen aus Steamcrackern oder katalytischen Crackern wird prinzipiell beschrieben in K.-D. Wiese, F. Nierlich, DGMK-Tagungsbericht 2004-3, ISBN 3-936418-23-3. Eine ausführliche Gesamtprozessbeschreibung findet sich in DE102008007081A1.

Die für diese Erfindung relevanten Aspekte der C₄-Aufarbeitung werden im Folgenden kurz umrissen.

Technische C₄- Kohlenwasserstoffgemische aus den oben beschriebenen Quellen enthalten üblicherweise neben gesättigten und einfach ungesättigten Verbindungen auch mehrfach ungesättigte Verbindungen. Bevor einzelne Verbindungen aus diesen Gemischen isoliert werden können, ist es häufig notwendig, andere Verbindungen möglichst vollständig zu entfernen. Dies kann durch physikalische Methoden, wie z. B. Destillation, Extraktivdestillation oder Extraktion, aber auch durch eine selektive chemische Umsetzung der zu entfernenden Komponenten erfolgen. Besonderes Augenmerk muss dabei auf der möglichst vollständigen Entfernung von den im C₄-Kohlenwasserstoffgemisch enthaltenden Verunreinigungen, wie Sauerstoff-, Stickstoff- und Schwefelhaltigen Komponenten liegen, da diese als Katalysatorgifte negative Auswirkungen auf die einzelnen Prozessschritte haben können. Während diese Verunreinigungen typischerweise in Crack C4 nur in Spuren vorhanden sind, können diese z.B. in FCC C4-Strömen auch in höheren Konzentrationen vorhanden sein.

C₄-Kohlenwasserstoffgemische aus Steamcrackern oder Fluidized Catalytic Crackern weisen typischerweise die in Tabelle 0 aufgeführten Hauptkomponenten auf (Verunreinigungen nicht dargestellt).

**Tabelle 0: Typische Zusammensetzungen von Crack C4 und FCC C4**

| **Komponente** | **Crack C4** | **FCC C4** |
|---|---|---|
| | [Gew.-%] | [Gew.-%] |
| Isobutan | 1 - 3 | 15 - 45 |
| n-Butan | 6 - 11 | 5 - 15 |
| 1-Buten | 14 - 20 | 5 - 20 |
| 2-Butene | 4 - 8 | 20 - 35 |
| Isobuten | 20 - 28 | 10 - 20 |
| 1,3-Butadien | 40 - 45 | kleiner 1 |

Die Zusammensetzung der Rohstoffe kann je nach Herkunft des Materials stark schwanken. Zu den aufgeführten Cα-Komponenten gesellen sich noch Kohlenwasserstoffe mit weniger oder mehr Kohlenstoffatomen, sowie Verunreinigungen wie Mercaptane, Sulfide, Disulfide, Stickstoff- und sauerstoffhaltige Verbindungen in geringen Mengen.

Die Aufarbeitung von FCC C4 kann in einer Variante so erfolgen, dass zunächst die Konzentration des Isobutans mittels eines destillativen Schrittes in einer Destillation auf einen Wert von kleiner 5 Gew.-% gesenkt wird. Gleichzeitig werden die im Gemisch vorhandenen Leichtsieder (zum Beispiel C₃-Kohlenwasserstoffe, leichte Sauerstoff, Stickstoff- und Schwefel enthaltende Verbindungen) entfernt bzw. minimiert. Im darauf folgenden Schritt werden in einer Kolonne alle Hochsieder (zum Beispiel C₅-Kohlenwasserstoffe, schwere Sauerstoff-, Stickstoff- und Schwefel enthaltende Verbindungen) über den Sumpf entfernt. Im nächsten Schritt wird Isobuten entfernt, z. B. indem es mit Methanol zu Methyl-tert.-butylether (MTBE) umgesetzt und dieser durch Destillation entfernt wird. Soll reines Isobuten gewonnen werden, kann der Methyl-tert.-butylether anschließend wieder zu Isobuten und Methanol gespalten werden.

Zur weiteren Aufarbeitung des C₄-Gemisches müssen die noch verbliebenen mehrfach ungesättigten Verbindungen mit Hilfe eines Selektivhydrierprozesses zu den entsprechenden einfach ungesättigten und gesättigten Verbindungen umgesetzt werden. Jetzt können 1-Buten und verbliebenes Isobutan in ausreichender Reinheit destillativ abgetrennt und die verbleibenden 2-Butene und das n-Butan weiter aufgearbeitet werden. Häufig werden die 2-Butene durch Oligomerisierung, genauer gesagt, durch Dimerisierung zu Oktenen umgesetzt. Dabei wird aus zwei Molekülen mit je vier Kohlenstoffatomen ein Molekül mit acht Kohlenstoffatome aufgebaut. Die Oktene können anschließend mittels Hydroformylierung zu PVC-Weichmacheralkoholen umgesetzt werden. Die nach Abreaktion der Olefine verbleibenden gesättigten C4-Kohlenwasserstoffe können insbesondere als Treibmittel für Aerosole verwendet werden.

Unter einer Oligomerisierung ist ein Prozess zu verstehen, bei dem aus Olefinen wie insbesondere aus Propen und Butenen, höhere Alkene mit 6-20 Kohlenstoffatomen aufgebaut werden. Industriell angewendet wird beispielsweise der Nickel-katalysierte OCTOL - Prozess, welcher in Hydrocarbon Process., Int. Ed. (1986) 65 (2. Sect.1), Seiten 31 bis 33 sowie in DE3914817, EP1029839 und DE102004018753 näher beschrieben ist. Die Oligomerisierung erfolgt nach dem OCTOL-Prozess in der Flüssigphase weswegen eine hohe Prozessintensität erreicht wird.

Die für die einzelnen Verfahrensschritte verwendeten Einsatzströme haben durch vorangehende Prozesse, in denen immer wieder Verunreinigungen entfernt wurden, in der Regel bereits einen hohen Grad an Reinheit erreicht. Verbliebene Verunreinigungen können jedoch die Katalysatoren reversibel oder auch irreversibel desaktivieren. Diese Desaktivierung soll aus wirtschaftlichen Gründen natürlich auf ein Minimum reduziert werden. Daher sollten so viele Katalysatorgifte wie möglich durch weitere Reinigungsstufen vom Katalysator ferngehalten werden.

Die verschiedenen in den technischen C₄-Gemischen vorhandenen Katalysatorgifte wirken in unterschiedlicher Weise vergiftend. So werden die sauren Katalysatorsysteme oder Systemkomponenten wie Cokatalysatoren fast ausschließlich durch Komponenten vergiftet, die selbst basisch sind oder zumindest durch Folgereaktionen Basen freisetzen. Ein besonders typisches Beispiel für solche Stoffe ist Acetonitril, das als sehr schwache Base vergleichsweise schwer durch Sorptionsprozesse abgetrennt werden kann. Es vergiftet jedoch reversibel starke Lewis-Säuren. In Anwesenheit von Spuren von Wasser hydrolysiert es dort über Acetamid zur starken Base Ammoniak, das dann auch Brönsted-Säuren durch Bildung von Ammoniumionen irreversibel desaktiviert. Ein partielles Katalysatorgift stellt im Übrigen immer auch Wasser selbst dar, dessen Wirkung jedoch in der Regel reversibel ist, sofern es nicht durch weitere Reaktionen zur Bildung stärkerer Katalysatorgifte beiträgt. Für die Nickel-katalysierte Oligomerisierung von Butenen am OCTOL-Katalysator führt bereits ein Wassergehalt von ca. 5 ppm zu messbarer Desaktivierung. Das Wasser wird jedoch von vielen Systeme an Olefine addiert und die gebildeten Alkohole werden durch die üblichen Katalysatorsysteme über eine Transferhydrierung unter Hydrierung von anderen ungesättigten Komponenten so lange oxidiert, bis das thermodynamische Gleichgewicht erreicht ist.

Auch die Metallkomplexkatalysatoren sind gegen basische Substanzen empfindlich. Die Vergiftungswirkung erfolgt dabei primär zumeist über die Desaktivierung des sauren Cokatalysators.

Die Metallkomponente der Katalysatoren wird hingegen besonders stark durch Komponenten wie Schwefel in Form bestimmter Verbindungen angegriffen, der unter bestimmten Umständen das Metallhydrid oder den Metallkomplex durch Bildung schwerlöslicher Sulfide irreversibel zerstört. Da die Metalle in der Regel in sehr niedrigen Oxidationsstufen vorliegen, sind besonders wirkungsvoll Schwefelverbindungen, die die Metalle zu einer relativ hohen Oxidationsstufe zu oxidieren vermögen, wie zum Beispiel Di- und Polysulfide. Unterschiedliche Schwefelverbindungen vermögen also primär durchaus unterschiedlich zu wirken. Während zum Beispiel Disulfide extrem gut abreagieren zu Thioethern und Schwefel, der dann die Metallhydride unter Bildung von Sulfiden oxidiert, wirken Thioether selbst primär zunächst wohl nur als Lewis-Base. Durch weitere, in der Regel im Detail gar nicht bekannte Prozesse und Reaktionen mit weiteren Spurenkomponenten des Systems führen auch sie aber letztlich auch - wenn auch wesentlich langsamer - zur Bildung von Metallsulfiden.

Nach dem vorangehend gesagten ist also für einen wirtschaftlich erfolgreichen Betrieb einer Anlage zur Zerlegung von Kohlenwasserstoffgemischen in seine Wertbestandteile mit Hilfe katalytischer Reaktionseinheiten die Aufgabe gestellt, die eingesetzten Katalysatoren möglichst effektiv vor Katalysatorgiften und insbesondere vor Schwefelverbindungen zu schützen. Dies trifft umso stärker zu, je mehr Edukt der Katalysator spezifisch umsetzen soll, in besonderem Maße also auf heterogene Katalysatoren wie beispielsweise den des OCTOL-Prozesses.

In der betrieblichen Praxis entfernen alkalische Wäschen schwefelhaltige Giftstoffe aus Propen- und Butenströmen. Dabei reagieren Schwefelwasserstoff und Mercaptane besonders gut. In der Regel werden die alkalischen Waschlösungen durch Oxidation mit Luft regeneriert.

Ein solches Waschverfahren wird für den industriellen Einsatz von der Fa. UOP LLC unter dem Namen MEROX^{®} angeboten. (G. A. Dziabis, "UOP MEROX PROCESS" in Robert Meyers, Handbook of Petroleum Refining Processes, 3rd Edition, 2004 McGraw-Hill).

Beim MEROX^{®}-Verfahren werden die Mercaptane in der wässrigen Waschlösung zu Di- und Polysulfiden oxidiert, die als ölige Phase abgetrennt werden. Ein kleiner Teil dieser Di- und Polysulfide verbleibt jedoch in der wässrigen Alkalilauge gelöst oder suspendiert, und es gelingt auch durch eine Wäsche dieser wässrigen Phase mit einem Waschöl oder ähnlichem oft nicht, diesen Rest vor der Rückführung in die Wäsche quantitativ zu entfernen, so dass zwar die Mercaptane weitgehend entfernt, auf der anderen Seite aber kleine Mengen an Di- und Polysulfiden wieder in den Strom eingetragen werden. Diese stellen, wie eben erwähnt, Schwefelkomponenten dar, die die für die Reaktion wesentlichen Metallhydride in schwerlösliche Metallsulfide umwandeln und den Katalysator dadurch irreversibel desaktivieren. Typischerweise enthalten zum Beispiel die Ströme an FCC C4 etwa 100 bis 200 ppm Schwefel. Nach der MEROX^{®}-Wäsche ist dieser Gehalt dann üblicherweise bis auf einen Wert von unter 10 ppm reduziert, wobei die Schwefelverbindungen dann überwiegend aus den genannten Di- und Polysulfiden, aber auch aus höheren Mercaptanen bestehen.

In der Praxis kann man einen Teil der Gifte auch durch geschickte Anordnung von Trennoperationen, wie zum Beispiel Destillationen, in Fraktionen lenken, in denen sie nicht mehr mit empfindlichen Katalysatoren in Berührung kommen. Häufig ist dies aber nicht in dem Umfange möglich, der in Hinblick auf die Reinheit der Ströme wünschenswert erscheint, so dass vor den Katalysatoren Sorptionsmittel vorgeschaltet werden müssen, um die erforderliche Reinheit zu gewährleisten.

Sorptionsmittel sind feste Stoffe, die in der Lage sind, einen anderen Stoff, das so genannte Sorbat, an sich zu binden, sofern sie mit dem Sorbat in Kontakt kommen. Die Bindung erfolgt an der Oberfläche des Sorptionsmittels durch physikalische und/oder chemische Effekte. Insoweit unterscheidet man physikalische und chemische Adsorption. Da die Wirkweise eines Sorptionsmittels nicht immer zweifelsfrei feststeht, wird hier wirkungsneutral von einem Sorptionsmittel gesprochen.

Aus technischer Sicht sind Sorptionsmittel allgemein zu unterscheiden in solche, die regenerierbar sind und in solche, die die Katalysatorgifte irreversibel umwandeln bzw. chemisch binden.

Als regenerierbare Sorptionsmittel kommen häufig Molekularsiebe und Zeolithe zum Einsatz. Die regenerierbaren Sorptionsmittel binden Verschmutzungen mit nur moderater Festigkeit. Im Zuge der Regenerierung des Sorptionsmittels werden Bedingungen wie zum Beispiel höheren Temperaturen und niedrigere Drücke eingestellt, unter denen das Sorptionsmittel das Sorbat wieder frei lässt. Diese Eigenschaft führt zu einer relativ geringen Kapazität bis zum Durchbruch. Zusätzlich entstehen oft hohe Betriebskosten durch Freistellen und Spülen des Sorptionsmittels sowie durch die Bereitstellung und Entsorgung der Regeneriergase oder auch der Flüssigkeitsströme.

Irreversible Sorptionsmittel werden dagegen nicht regeneriert sondern nach ihrem Durchbruch entsorgt. Sie müssen daher preiswert verfügbar und entsorgbar sein. Da irreversiblen Sorptionsmittel das Adsorbat chemisch binden, ist ihre Durchlässigkeit gegenüber den zu adsorbierenden Stoffen geringer als bei regenerierbaren Sorptionsmitteln. Irreversible Sorptionsmittel erzielen deswegen bessere Reinheitsgrade als regenerierbare Sorptionsmittel.

EP 0 064 464 A1 beschreibt Kontaktmassen, welche insbesondere zur Entschwefelung von Kohlenwasserstoffchargen verwendbar sind. Die Kontaktmassen enthalten Kupferoxid und basieren auf einem Träger aus Tonerde oder Zeolith von Typ X oder Y. Bedenklich ist der zwingend erforderliche Gehalt an Cadmiumoxid, da Cadmium als kanzerogen eingestuft ist. Kanzerogene Stoffe lassen sich nur aufwendig handhaben und entsorgen, sodass insbesondere der irreversible Einsatz derartiger Kontaktmassen unwirtschaftlich ist.

EP 0 354 316 B1 beschreibt die cadmiumfreie Feinentschwefelung von flüssigen C₄-Kohlenwasserstoffgemischen an Kupfer, Silber und Zink enthaltenden Zeolithen. Der bevorzugte Temperaturbereich liegt zwischen 50 und 130°C, der bevorzugte Druck bei 1 bis 50 bar. Die Raum/Zeit-Belastung wird mit 1 bis 40 h⁻¹ angegeben. Wenngleich das hier beschriebene Sorptionsmittel kein bedenkliches Cadmium enthält, ist dieses Material aufgrund seines hohen Silbergehalts von mindestens 2 Gew.-% ebenso unwirtschaftlich.

Besonders anfällig gegen Katalysatorgifte sind Nickel-haltige Oligomerisierungskatalysatoren. Kohlenwasserstoffgemische mit zwei bis vier Kohlenstoffatomen dienen oft als Substrat für Oligomerisierungen wie dem OCTOL-Prozess. Um Katalysatorgifte effektiv zu entfernen hat sich bewährt, solche Ströme vor dem Eintritt in die Oligomerisierung über ein Molekularsieb zu leiten. So beschreibt etwa EP0395857B1 ein Verfahren, bei welchem eine Entschwefelung von Raffineriepropen vor dessen Oligomerisierung an einem mit Kupfer ausgetauschten X-Zeolithen bei einer Temperatur von 120°C, einem Druck von 50 bar abs. und einer Raum/Zeit-Belastung von 0.75 h⁻¹ erfolgt. Bei diesen Bedingungen ist Propen überkritisch.

Da diese einfachen Molekularsiebe leicht verfügbar und gesundheitlich unbedenklich sind, stellen sie in der industriellen Praxis heute das Sorptionsmittel der Wahl zur Feinentschwefelung von C₃ - bis C₈ - Kohlenwasserstoffgemischen dar. Da unmodifizierte Molekularsiebe die Verunreinigungen im Wesentlichen physikalisch binden, lassen sich derartige Sorptionsmittel regenerieren. Allerdings ist ihr Sorptionsvermögen im Vergleich zu chemisch wirkenden Sorptionsmitteln geringer, sodass mit der Feinentschwefelung an unmodifizierten Zeolithen nur mäßige Reinheiten zu erzielen sind. Um diesen Nachteil auszugleichen, werden Zeolithe dergestalt modifiziert, dass sie Verunreinigungen auch chemisch arretieren; dies schränkt aber wiederum die Regenerierbarkeit der modifizierten Zeolithe ein.

Aus der WO2014/009159A1 ist die Verwendung von pyrophorem Nickel zur irreversiblen Schwefeladsorption vor Oligomerisierungsanlagen bekannt. In einem Versuch wird dieses Sorptionsmittel erfolgreich zur Entfernung von Dimethylsulfid aus einem C₄-Olefingemisch genutzt. Nachteil dieses Sorptionsmittels sind seine pyrophoren Eigenschaften, die seine Handhabung erschweren. Der industrielle Einsatz ist daher nur unter Einschränkungen möglich.

In der zum Anmeldezeitpunkt noch unveröffentlichten deutschen Patentanmeldung 102013225724.4 ist die Reinigung flüssiger Olefingemische mittels eines Kupfer/Zink/Aluminiumkatalysators beschrieben, der normalerweise in der Methanolsynthese eingesetzt wird. Die Reinigung findet in Abwesenheit von Wasserstoff statt. Versuche belegen, dass dieses Material alle üblicherweise in C4-Olefingemischen vorkommenden Schwefelverbindungen (insbesondere Mercaptane) nahezu vollständig bindet. Gegenüber dem pyrophoren Nickelmaterial hat es den Vorteil, dass es sich einfacher handhaben lässt.

Ein Nachteil dieses CuO/ZnO/Al₂O₃-Sorptionsmittel ist, dass es einen Teil des eingetragenen Schwefels in Form von Disulfiden, genauer gesagt Dimethyldisulfid, Diethyldisulfid, Ethylmethyldisulfid und ähnliche Substanzen wieder abgibt. Die Disulfid-Bildung findet im Sorptionsmittel selbst statt; das Material erscheint insoweit katalytisch aktiv: Offensichtllich werden auf der CuO-Oberfläche zwei Thiolat-Einheiten oxidativ zu Disulfiden verknüpft. Dies geschieht freilich nur in geringem Maße.

Ein bedeutender Nachteil der in DE102013225724 beschriebenen CuO/ZnO/Al₂O₃-Sorptionsmittel ist die geringe Adsorptionskapazität für Schwefel bzw. schwefelhaltige Verbindungen in Höhe von etwa nur 1.4 Gew-%. Im Vergleich dazu verspricht WO2014/009159A1 eine Schwefelkapazität von etwa 25 %.

Aus der WO94/28089 ist die Verwendung von elementarem Cu enthaltenden Adsorbern zur irreversiblen Schwefeladsorption bekannt. Diese werden aus CuO-haltigen Vorstufen durch vorhergehende Reduktion des CuO mit Wasserstoff zu elementarem Cu gewonnen. Es wird dort beschrieben, dass elementares Cu deutlich reaktiver gegenüber Mercaptanen und elementarem Schwefel sei als CuO. Disulfide werden offensichtlich nicht gut über solchen Materialien zurückgehalten. Ein weiterer Nachteil ist die notwendige Reduktion des CuO im Wasserstoffstrom zu elementarem Cu bei hohen Temperaturen vor seinem Einsatz in einem Adsorptionsreaktor. Die erfordert entweder einen teuren, temperaturbeständigen Reaktor oder eine ex-situ Konditionierung mit anschließendem Einbau des Adsorptionsmittels unter Schutzatmosphäre.

Auch die EP0320979A2 beschreibt Entschwefelungsmittel auf Basis von Kupfer-, Zink- und Aluminiumoxid. Allerdings wird das oxidische Sorptionsmittel vor seinem Einsatz mit Wasserstoff reduziert, sodass es letztendlich metallisch eingesetzt wird.

Aus US2007034552 ist die Verwendung von CuO/ZnO/Al₂O₃ als schwefelhaltige Verbindungen abfangende Materialien bekannt. Es wurde anhand von Butanthiol als S-Komponente in Naphtha als Kohlenwasserstoffgemisch bei der Adsorption in flüssiger Phase gezeigt, dass zwischen 3,7 und 10 Gew.-% Schwefel (gerechnet in seiner elementaren Form) auf den verschiedenen CuO/ZnO/Al₂O₃-Materialien zurückgehalten werden können. Allerdings wurde als Zeitpunkt des Durchbruchs durch das Adsorptionsbett definiert, wenn 80% des zugefahrenen Schwefels im Austrag des Adsorbers detektiert werden. Dies bedeutet, dass zu diesem Zeitpunkt die adsorbierende Wirkung faktisch schon verloren ist. Für nachgeschaltete Prozesse, die selbst auf einige Dutzend ppb Schwefel empfindlich reagieren, ist eine solche Durchbruchsdefinition absolut ungeeignet.

In W. Turbeville et al., Cat.Today, 519-525 (2006) werden zu US2007034552 analoge Materialien und ihre Adsorptionskinetik näher beschrieben. Aus den Graphen zum Schwefel-Durchbruch ist ersichtlich, dass vertretbare Durchbruchdefinitionen, wie z.B. ein Durchbruch bei 20% des Zulauf-Schwefel-Gehaltes, bei deutlich niedrigeren Laufzeiten auftreten. So liegt die Zeit bis zum Erreichen der 20%-Durchbruchsmarke bei allen gezeigten Versuchen bei rund einem Viertel der 80%-Marke. Dies bedeutet, dass im industriell relevanteren Anwendungsfalle einer 20%-Durchbruchsgrenze die Standzeit auf ein Viertel verkürzt ist, also der Austausch des Adsorbers viermal häufiger vorgenommen werden muss. Ein weiterer Nachteil der von Turbeville et al. beschriebenen Materialien und Versuche ist der dauerhafte Durchtritt von aus Mercaptanen gebildeten Disulfiden, die anscheinend nicht signifikant adsorbiert werden.

Die EP 1 192 981 A1 offenbart ein Verfahren zur Herstellung eines Entschwefelungsmittels aus CuO, ZnO und Al₂O₃. Weiterhin wird ein Verfahren zur Entschwefelung von Kohlenwasserstoffgemischen mittels eines Adsorptionsmittels, welches CuO, ZnO und Al₂O₃ enthält, offenbart, wobei das Verfahren sich durch einen nur geringen Einsatz des Entschwefelungsmittels auszeichnet.

Vor diesem Hintergrund lag der Erfindung die Aufgabe zu Grunde, ein Verfahren zur Reinigung von flüssigen Olefingemischen anzugeben, bei welchem im Gemisch vorhandene Schwefelverbindungen nahezu vollständig entfernt werden, ohne dabei wieder in signifikantem Maße neue Schwefelverbindungen zu bilden. Nach der Reinigung soll ein Schwefelgehalt von deutlich unter 1 ppm gewährleistet werden, sodass nachfolgende katalytische Prozesse wie insbesondere Oligomerisierungen nicht vergiftet werden. Als weitere wichtige Aufgabe dieser Erfindung soll ein Verfahren aufgezeigt werden, das zu einer deutlich erhöhten Kapazität bzw. Standzeit des eingesetzten Sorptionsmittels führt. Zudem soll bei der Reinigung des Gemisches darin enthaltende Wertprodukte wie zum Beispiel 1-Buten nicht verloren gehen, denn das Verfahren soll sich dazu eignen, auch solche Kohlenwasserstoffgemische zu reinigen, die einen hohen Anteil an wertvollem 1-Buten haben, der bei einem nicht geeigneten Sorptionsmaterial zu weniger wertvollen 2-Butenen isomerisiert würde. Schließlich soll das eingesetzte Sorptionsmittel möglichst frei von kanzerogenen Bestandteilen und einfach verfügbar sein.

Gelöst werden diese Aufgaben dadurch, dass ein Sorptionsmittel eingesetzt wird, welches die folgende, sich zu 100 Gew.-% ergänzende Zusammensetzung aufweist:
- Kupferoxid: 10 Gew.-% bis 60 Gew.-% (gerechnet als CuO);
- Zinkoxid: 10 Gew.-% bis 60 Gew.-%, (gerechnet als ZnO);
- Aluminiumoxid: 10 Gew.-% bis 30 Gew.-% (gerechnet als Al2O3);
- sonstige Stoffe: 0 Gew.-% bis 5 Gew.-%;
und dass die Reinigung in Gegenwart Wasserstoff durchgeführt wird.

Es hat sich nämlich gezeigt, dass ein solches CuO/ZnO/Al2O3-System nämlich dann nahezu keine schwefelhaltigen Verbindungen jeglicher Art mehr abgibt, wenn während des Kontakts mit dem verunreinigten Kohlenwasserstoffgemisch geringe Mengen an Wasserstoff gegenwärtig sind. Die hervorragende Fähigkeit des Materials, Schwefelverbindungen wie beispielsweise Mercaptane zu binden, wird dabei durch die Anwesenheit des Wasserstoffs sogar noch verstärkt. Die Kapazität des Sorptionsmittels wird mithin gesteigert. Des Weiteren werden nun auch verstärkt Schwefelkomponenten gebunden, die nach dem bisherigen Kenntnisstand kaum auf entsprechenden Adsorbern zurückgehalten wurden. Da der Wasserstoff nur in geringen Mengen zugesetzt wird, bleiben reaktive Wertprodukte wie 1-Buten weitestgehend erhalten, denn sie gehen kaum durch unerwünschte Hydrierung und/oder Hydroisomerisierung verloren. Andere unerwünschte Nebenreaktionen der Wertprodukte werden durch das eingesetzte Sorptionsmaterial kaum begünstigt.

Gegenstand der Erfindung ist mithin ein Verfahren zur Reinigung von Kohlenwasserstoffgemischen, bei welchem ein verunreinigtes Kohlenwasserstoffgemisch enthaltend Olefine mit drei bis acht Kohlenstoffatomen durch Kontaktieren mit einem festen Sorptionsmittel in Gegenwart von Wasserstoff zumindest teilweise von schwefelhaltigen Verunreinigungen befreit wird, wobei sich das Kohlenwasserstoffgemisch während des Kontakts mit dem Sorptionsmittel ausschließlich im flüssigen Zustand befindet, und wobei das Sorptionsmittel die folgende, sich zu 100 Gew.-% ergänzende Zusammensetzung aufweist:
- Kupferoxid: 10 Gew.-% bis 60 Gew.-% (gerechnet als CuO);
- Zinkoxid: 10 Gew.-% bis 60 Gew.-%, (gerechnet als ZnO);
- Aluminiumoxid: 10 Gew.-% bis 30 Gew.-% (gerechnet als Al₂O₃);
- sonstige Stoffe: 0 Gew.-% bis 5 Gew.-%,

wobei der Kontakt des verunreinigten Kohlenwasserstoffgemisches mit dem festen Sorptionsmittel in Gegenwart von Wasserstoff erfolgt, wobei das verunreinigte Kohlenwasserstoffgemisch unmittelbar vor dem Kontakt mit dem festen Sorptionsmittel Wasserstoff enthält in einer Konzentration, die auf die Gesamtmasse des verunreinigten Kohlenwasserstoffgemisches bezogen zwischen 1 Gew.-ppm und 10000 Gew.-ppm beträgt, wobei besagte Konzentration an Wasserstoff dadurch eingestellt wird, dass dem verunreinigten Kohlenwasserstoffgemisch unmittelbar vor dem Kontakt Wasserstoff zugegeben wird, wohingegen das verunreinigte Kohlenwasserstoffgemisch vor Zugabe des Wasserstoffs weniger als 1 Gew.-ppm Wasserstoff enthält,
dass das verunreinigte Kohlenwasserstoffgemisch 1-Buten enthält, und dass durch den Kontakt mit dem Sorptionsmittel weniger als 5 % des im verunreinigten Kohlenwasserstoffgemisch enthaltenden 1-Buten umgesetzt wird, und
dass das verunreinigte Kohlenwasserstoffgemisch eine der folgenden, sich jeweils zu 100 Gew.-% ergänzende Spezifikationen A, B, C oder D erfüllt, wobei sich die angegebenen Gewichtsanteile jeweils auf das Gesamtgewicht des verunreinigten Kohlenwasserstoffgemisches beziehen.
   Spezifikation A:
      - Isobutan 15 bis 45 Gew.-%, bevorzugt 25 bis 35 Gew.-%;
      - n-Butan 5 bis 18 Gew.-%, bevorzugt 8 bis 10 Gew.-%;
      - 1-Buten 5 bis 20 Gew.-%, bevorzugt 12 bis 14 Gew.-%;
      - Isobuten 12 bis 25 Gew.-%, bevorzugt 15 bis 20 Gew.-%;
      - 2-Butene 9 bis 40 Gew.-%, bevorzugt 20 bis 30 Gew.-%;
      - 1.3-Butadien 0 bis 3 Gew.-%, bevorzugt 0.5 bis 0.8 Gew.-%;
      - Wasser 0 bis 1 Gew.-%, bevorzugt weniger als 0.1 Gew.-%;
      - schwefelhaltige Verunreinigungen weniger als 0.5 Gew.-%, bevorzugt unter 0.2 Gew.-%.
      - Wasserstoff weniger als 1 Gew.-ppm.
   Spezifikation B:
      - Isobutan 0.5 bis 15 Gew.-%, bevorzugt 1 bis 7 Gew.-%;
      - n-Butan 0.5 bis 20 Gew.-%, bevorzugt 4 bis 7 Gew.-%;
      - 1-Buten 9 bis 25 Gew.-%, bevorzugt 10 bis 20 Gew.-%;
      - Isobuten 10 bis 35 Gew.-%, bevorzugt 20 bis 30 Gew.-%;
      - 2-Butene 3 bis 15 Gew.-%, bevorzugt 5 bis 10 Gew.-%;
      - 1.3-Butadien 25 bis 70 Gew.-%, bevorzugt 40 bis 50 Gew.-%;
      - Wasser 0 bis 1 Gew.-%, bevorzugt weniger als 0.5 Gew.-%;
      - schwefelhaltige Verunreinigungen weniger als 0.5 Gew.-%, bevorzugt unter 0.2 Gew.-%.
      - Wasserstoff weniger als 1 Gew.-ppm.
   Spezifikation C:
      - Isobutan 0.5 bis 18 Gew.-%, bevorzugt 1 bis 7 Gew.-%;
      - n-Butan 0.5 bis 25 Gew.-%, bevorzugt 4 bis 13 Gew.-%;
      - 1-Buten 9 bis 40 Gew.-%, bevorzugt 10 bis 35 Gew.-%;
      - Isobuten 10 bis 55 Gew.-%, bevorzugt 20 bis 50 Gew.-%;
      - 2-Butene 3 bis 25 Gew.-%, bevorzugt 5 bis 20 Gew.-%;
      - 1.3-Butadien 0 bis 5 Gew.-%, bevorzugt weniger als 0.8 Gew.-%;
      - Wasser 0 bis 1 Gew.-%, bevorzugt weniger als 0.5 Gew.-%;
      - schwefelhaltige Verunreinigungen weniger als 0.5 Gew.-%, bevorzugt unter 0.2 Gew.-%.
      - Wasserstoff weniger als 1 Gew.-ppm.
   Spezifikation D:
      - Isobutan 0 bis 20 Gew.-%, bevorzugt 0 bis 5 Gew.-%;
      - n-Butan 10 bis 35 Gew.-%, bevorzugt 25 bis 30 Gew.-%;
      - 1-Buten 0.2 bis 45 Gew.-%, bevorzugt 3 bis 30 Gew.-%;
      - 2-Butene 35 bis 85 Gew.-%, bevorzugt 50 bis 75 Gew.-%;
      - Wasser 0 bis 1 Gew.-%, bevorzugt weniger als 0.1 Gew.-%;
      - schwefelhaltige Verunreinigungen weniger als 0.5 Gew.-%, bevorzugt unter 0.1 Gew.-%.
      - Wasserstoff weniger als 1 Gew.-ppm.

Ein praktischer Vorteil des erfindungsgemäßen Verfahrens besteht darin, dass besagtes CuO/ZnO/Al₂O₃-System nicht eigens hergestellt werden muss sondern einfach kommerziell verfügbar ist, nämlich als Katalysator, wie er normalerweise in der Methanolsynthese eingesetzt wird.

Methanol ist eine bedeutende Grundchemikalie, die aus einem Gasgemisch aus Wasserstoff, Kohlenmonoxid und Kohlendioxid in Gegenwart von festen Kupfer/Zink/Aluminiumkatalysatoren synthetisiert wird. Da Methanol in weltweit in sehr großen Mengen hergestellt wird, sind die dafür benötigten Kupfer/Zink/Aluminiumkatalysatoren leicht verfügbar. Ein wesentlicher Aspekt der Erfindung besteht darin, solche Methanol-Katalysatoren als Sorptionsmittel zur Entschwefelung von Olefingemischen in Gegenwart von Wasserstoff zu nutzen.

Feste Kupfer/Zink/Aluminiumkatalysatoren für die Methanolsythese sind in der Patentliteratur vielfach beschrieben:
So offenbart DE2846614C3 ein Verfahren zur Herstellung von Methanol aus einem Gasgemisch von CO, CO₂ und H₂ bei Temperaturen von 200 bis 350°C in Gegenwart eines Katalysators, der 38.3 % Cu, 48.8 % Zn und 12.9 % Al enthält.

DE1568864C3 weist darauf hin, dass Synthesegas für die Methanolherstellung entschwefelt werden sollte, da sich kupferhaltige Katalysatoren mit Schwefel leicht vergiften lassen. Der hier beschriebene Kupfer/Zink/Aluminiumkatalysator enthält mehr als 35 Gew.-% Kupfer, der Zinkgehalt beträgt 15 bis 50 Gew.-%. Der Aluminiumgehalt wird mit 4 bis 20 Gew. -% angegeben.

EP0125689B2 beschreibt einen Katalysator für die Methanolsynthese, welcher als katalytisch wirksame Substanzen Kupferoxid und Zinkoxid enthält, sowie - als thermostabilisierende Substanz - Aluminiumoxid. Im nicht reduzierten Zustand weisen beispielhaft hergestellte Katalysatorvorstufen etwa 65 bis 68 Gew.-% CuO, 21 bis 23 Gew.-% ZnO und 10 bis 12 Gew.-% Al₂O₃ auf. Die spezifische Oberfläche beträgt 100 bis 130 g/m². Die Methanolsynthese erfolgt bei 250°C und 50 bar.

Ähnliche Methanolkatalysatoren mit 63 bis 65 Gew.-% CuO, 24 bis 27 Gew.-% ZnO und 10 bis 11 Gew.-% Al₂O₃ sind in der DE10160486A1 beschrieben.

Ein Katalysator mit vergleichsweise geringen Kupfer- und hohem Zink-Gehalt (43.2 Gew.-% CuO, 47.0 Gew.-% ZnO und 10.2 Gew.-% Al₂O₃ ) wurde in US4279781 hergestellt. Dessen katalytische Aktivität in der Methanolsynthese wurde allerdings als eher schlecht bewertet.

Mit der Herstellung von Kupfer-, Zink-, Aluminiumoxid-Katalysatoren für die Methanol-Synthese befassen sich wissenschaftlich R.H. Höppener, E.B.M. Doesburg, J.J.F. Scholten: Preparation and characterization of stable copper/zinc oxide/alumina catalysts for methanol systhesis. Appl. Catal. 25 (1986) 109-119.

Aufgrund der großen industriellen Bedeutung der Synthese der Grundchemikalie Methanol sind Kupfer/Zink/Aluminiumkatalysatoren nicht nur theoretisch in der wissenschaftlichen Literatur und in Patentliteratur beschrieben sondern auch kommerziell leicht verfügbar. Zu nennen sind beispielsweise MegaMax^{®} 700 und 800 von Clariant (vormals Süd-Chemie) und Haldor Topsoe's Mk-101 und Mk-121.

Die Entsorgung von diesem Material ist vergleichsweise unproblematisch, da keine als kanzerogen eingestuften Stoffe enthalten sind. Im Übrigen ist das Recycling solcher Sorptionsmittel wirtschaftlich attraktiv, da dieses Material viel wertvolles Kupfer enthält.

Die Eignung eines Methanol-Katalysators zur Entschwefelung von C₃ - bis C₈-Kohlenwasserstoffgemischen ist deswegen verwunderlich, weil die Aufarbeitung solcher Gemische in der Regel in der Flüssigphase erfolgt, da sich die Kohlenwasserstoffe mit mehr als zwei Kohlenstoffatomen mit geringem Aufwand verflüssigen und dann mit einer hohen Prozessintensität verarbeiten lassen. Die Methanol-Synthese indes erfolgt ausschließlich in der Gasphase. Es war nicht zu erwarten, dass für die Gasphasenkatalyse bestimmten Materialen sich auch zur Flüssigphasensorption eignen würden.

Grundsätzlich eignet sich jeder kommerziell verfügbare Cu/Zn/Al-Katalysator mit genannter Zusammensetzung als festes Sorptionsmittel zur Reinigung der C₃- bis C₈-Kohlenwasserstoffgemischen in Gegenwart von Wasserstoff. Bevorzugt werden jedoch solche Katalysatoren eingesetzt, welche die folgende Zusammensetzung aufweisen:
- Kupferoxid: 30 bis 45 Gew.-% (gerechnet als CuO);
- Zinkoxid: 30 bis 50 Gew.-%, (gerechnet als ZnO);
- Aluminiumoxid: 10 bis 15 Gew.-% (gerechnet als Al₂O₃);
- weitere Metalloxide: 0 bis 2 Gew.-%;
- Graphit: 0 bis 3 Gew.-%;
- andere Stoffe: 0 bis 1 Gew.-%.

Als weitere Metalloxide kommen in diesem Zusammenhang beispielsweise Eisenoxide oder Magnesiumoxide in Betracht. Schwermetalloxide, die bekanntermaßen gesundheitsgefährdend sind, wie beispielsweise Cadmium oder Blei oder Chrom, sollten tunlichst nicht enthalten sein. Geringere Mengen an Graphit oder Magnesiumstearat dienen als Bindemittel zur besseren Formgebung des Sorptionsmittels. Unter "anderen Stoffen" sind in diesem Zusammenhang produktionsbedingte Verunreinigungen des Sorptionsmittels zu verstehen.

Hinsichtlich der Formgebung kann das Sorptionsmittel in Pulverform oder als Granulat vorliegen. Darüber hinaus kann das Sorptionsmittel in eine definierte Form gepresst sein, wie beispielsweise in Kugeln, Pellets, Tabletten, Ringe, Tori oder trilobulare Formkörper.

Die Verwendung von Material mit einer großen Kupferoxidoberfläche ist vorteilhaft, weil die Reaktionsgeschwindigkeit der Adsorption und der Umwandlung mit ihr korreliert und diese Materialien auch eine höhere Sorptionskapazität aufweisen. Bevorzugt weist das erste Sorptionsmittel eine Kupferoxidoberfläche von mindestens 50 m²/g, bevorzugt 100 m²/g, bezogen auf seinen Kupferoxidgehalt auf. Dies begünstigt die Sorptionswirkung. Die Oberfläche wird per Stickstoff-Sorption bestimmt.

Die Herstellung des Sorptionsmittels geeignet sind grundsätzlich alle technischen Methoden, die zu einem Festkörper führen, welcher eine ausreichende Festigkeit zur Handhabung besitzt. Sie umfasst im Wesentlichen die beiden Arbeitsschritte:
i) Bereitstellen eines porösen Gerüstmaterials aus Aluminiumoxid;
ii) Vermengen des Gerüstmaterials mit Kupferoxid und Zinkoxid.

Eingesetzt werden können Kupferoxidpulver, Kupfercarbonatpulver oder hydroxidhaltige Kupferverbindungen sowie Gemische daraus. Im Falle des Kupfers kann auch eine kupfercarbonathaltige Verbindung mit Hilfe einer ammoniakalischen Lösung ganz oder teilweise in eine Kupfertetramincarbonatlösung überführt werden, die als Einsatzmaterial dient. Diese Substanzen werden entsprechend den erfindungsgemäßen Mischungsverhältnissen zusammen mit Zinkoxid, Zinkcarbonat oder Zinkhydroxid sowie einem Al₂O₃-haltigem Pulver vermischt. Dieses Pulver dient als Gerüstmaterial. Als Al₂O₃-haltiges Pulver können alle Modifikationen von Al₂O₃ eingesetzt werden sowie auch Aluminiumoxidhydrat oder Aluminiumhydroxyoxide sowie Aluminiumhydroxid. Es kann neben Al₂O₃ auch teilweise SiO₂ enthalten. Die einzelnen Feststoffkomponenten können in geeigneten Mischern, Intensivmischern oder Knetern vermengt und homogenisiert werden. Dabei ist es üblich, eine Befeuchtung mit entmineralisiertem Wasser vorzunehmen. Nach ausreichender Mischung kann eine beliebig geeignete Formgebung erfolgen. Unter Umständen ist eine vorhergehende ganz oder teilweise Trockung und/oder Mahlung der Mischung nötig. Für die Formgebung sind beispielsweise Extruder oder Tablettenpressen geeignet. Auch Pelletierteller können für diese Zwecke dienlich sein. Im Falle einer Tablettierung wird dem Gemisch oftmals ein Gleithilfsmittel wie Graphit zugeführt. Im Falle einer Extrusion werden oft andere organische Zusätze gewählt, die geeignet sind die notwendige Plastifizierbarkeit des Gemisches einzustellen. Dazu zählen beispielsweise zelluloseartige Substanzen, Polyether, Polyethylenglycol und andere, die unter Umständen auch als Porenbildner dienen können, wenn die Substanzen durch eine thermische Behandlung, die sich der Formgebung in der Regel anschließt, ganz oder teilweise entfernt werden. Im Falle einer Pelletierung auf einem entsprechenden Pelletierteller wird die Aufbauagglomeration durch die langsame Zugabe einer geeigneten Menge an Wasser erreicht. Die Zugabe von Magnesiumstearat hilft bei der Verfestigung des Pulvers zu definierten Formkörpern.

Die thermische Behandlung wird in einem Schritt oder in sequentiellen Schritten durchgeführt. Hierbei werden Wasseranteile oder aber organische Anteile entfernt und die mechanische Festigkeit des Formkörpers wird in der Regel dabei erhöht. Außerdem werden die notwendigen Oxidphasen ausgebildet, wenn die Vorstufenmaterialien noch nicht in dieser Form vorlagen.

In einer anderen Art der Herstellung werden Nitratsalze in wässriger Lösung eingesetzt oder die oxydischen Verbindungen werden mit Salpetersäure ganz oder teilweise gelöst. Insbesondere im Fall der aluminiumoxidischen Verbindungen löst man oftmals nicht vollständig auf sondern modifiziert das Material mit Hilfe der Säure, dieser Vorgang wird als Peptisierung bezeichnet. Das Peptid wird dann mit den anderen gelösten Komponenten wie oben beschreiben vermischt und zu einem Formkörper verarbeitet. Die Temperung führt dann dazu, dass sich aus den Nitraten die jeweiligen Oxide ausbilden können, wenn die Temperatur geeignet gewählt wurde.

Die Verwendung von nitrathaltigen Salzlösungen kann auch dazu führen, dass eine Fällungsreaktion durchgeführt werden muss, um zu einem Feststoffgemisch zu kommen. Die pH-Einstellung erfolgt mit Natronlauge- oder Sodalösungen. Beispiele hierzu finden sich in EP0125689B2.

Weiterhin ist es möglich, Nitratsalzlösungen mittels Sprühtrocknung in ein oxydisches Produktgemisch als Feststoff zu überführen. In der Regel folgen dann eine Mahlung und eine wie zuvor beschriebene Formgebung. Eine abschließende Temperung, die aber auch direkt nach der Sprühtrockung oder der Mahlung der Bestandteile durchgeführt werden kann, führt die notwendige restliche Nitratzersetzung herbei und überführt die Komponenten hinzu den Oxiden und verfestigt den Formköper.

Die vorstehend beschriebene Eigenfertigung des Sorptionsmittels kann durch Verwendung eines kommerziell verfügbaren Methanol-Katalysators entfallen.

Ein wesentlicher Aspekt der Erfindung besteht darin, dass die Reinigung, also der Kontakt des verunreinigten Kohlenwasserstoffgemisches mit dem Sorptionsmittel, in Gegenwart von Wasserstoff erfolgt. Insoweit unterscheidet sich die Erfindung von diskutierten Verfahren, die in Abwesenheit von Wasserstoff durchgeführt werden.

Unter Gegenwart von Wasserstoff soll ein Massengehalt an molekularem Wasserstoff (H₂) von über 1 ppm bezogen auf die Gesamtmasse des verunreinigten Kohlenwasserstoffgemisches zum Zeitpunkt des Kontakts verstanden werden. Die Maßeinheit ppm bedeutet hier stets 10⁻⁶.

Genauer gesagt, sollte das verunreinigte Kohlenwasserstoffgemisch unmittelbar vor dem Kontakt mit dem festen Sorptionsmittel Wasserstoff enthalten in einer Konzentration, die auf die Gesamtmasse des verunreinigten Kohlenwasserstoffgemisches bezogen zwischen 1 Gew.-ppm und 10000 Gew.-ppm beträgt. Ein höherer Wasserstoffgehalt führt nämlich zu einer unerwünschten Hydrierung oder Hydroisomerisierung von im Kohlenwasserstoffgemisch enthaltenden Wertprodukten, wie insbesondere 1-Buten.

Entscheidend ist, dass der Wasserstoff und das zu reinigende Kohlenwasserstoffgemisch gleichzeitig mit dem Sorptionsmittel in Kontakt kommen. Es genügt nicht, das Sorptionsmittel vor dem Kontakt mit dem Kohlenwasserstoffgemisch mit Wasserstoff zu behandeln; vielmehr muss der Wasserstoff in dem zu reinigenden Kohlenwasserstoffgemisch gelöst sein.

Als geeigneter Wasserstoffgehalt zum Zeitpunkt des Kontakts hat sich eine Konzentration von 1 bis 10000 ppm erwiesen; wiederum bezogen auf die Gesamtmasse des verunreinigten Kohlenwasserstoffgemisches.

Der Wasserstoff sollte möglichst vollständig in dem verunreinigten Kohlenwasserstoffgemisch gelöst sein. Dies bedeutet, dass die Reinigung in Abwesenheit von gasförmigem Wasserstoff erfolgt. Weil das verunreinigte Kohlenwasserstoffgemisch erfindungsgemäß flüssig ist, liegt zum Kontaktzeitpunkt gar keine störende Gasphase vor. Dies erhöht die Prozessintensität.

Da der homogen flüssig gelöste maximale Wasserstoffgehalt sowohl vom Betriebsdruck, als auch von der Betriebstemperatur abhängt, hat sich unter bevorzugten Betriebsbedingungen mit Temperaturen zwischen 10°C und 150°C und Drücken zwischen 0.5 und 3.5 MPa ein Wasserstoffgehalt von 1 Gew.-ppm bis 1000 Gew.-ppm als praktikabel erwiesen. Als bevorzugter Wasserstoffgehalt hat sich eine Konzentration von 10 Gew.-ppm bis 500 Gew.-ppm erwiesen, als besonders bevorzugter Wasserstoffgehalt zum Zeitpunkt des Kontakts hat sich eine Konzentration von 50 Gew.-ppm bis 300 Gew.-ppm erwiesen.

Technische Kohlenwasserstoffströme, für deren Reinigung das vorliegende Verfahren bestimmt ist, sind in der Regel frei von Wasserstoff; die Wasserstoffkonzentration üblicher C₃- und C₄-Olefingemische der Petro-Folgechemie liegt unter 1 Gew.-ppm.

Dies bedeutet, dass die erfindungsgemäße Konzentration an Wasserstoff dadurch eingestellt werden muss, dass dem verunreinigten Kohlenwasserstoffgemisch unmittelbar vor dem Kontakt Wasserstoff zugegeben wird, da ansonsten das verunreinigte Kohlenwasserstoffgemisch beim Kontakt weniger als 1 Gew.-ppm Wasserstoff enthalten würde.

Es ist somit eine technische Maßnahme erforderlich, welche die Zudosierung von Wasserstoff in das verunreinigte Kohlenwasserstoffgemisch gestattet. Dies kann ein handelsüblicher gas/flüssig-Mischer sein, da der Wasserstoff gasförmig in das flüssige Kohlenwasserstoffgemisch eingespeist wird. Aufgrund seiner geringen Konzentration wird der Wasserstoff vollständig in dem flüssigen Kohlenwasserstoffgemisch gelöst, sodass der Kontakt an dem Sorptionsmittel ein reiner flüssig/fest Kontakt ist, d.h. ohne Anwesenheit einer Gasphase.

Die Wasserstoffkonzentration in dem verunreinigten Kohlenwasserstoffgemisch lässt sich einfach durch einen geeignetes Fördergerät, wie einen Wasserstoff-Gasregler, einstellen. Da dies gängige Praxis in der chemischen Industrie ist, kann auf diese Weise ein hoher Automatisierungsgrad erreicht werden. Bei Bedarf kann aber einen eine gaschromatographische Bestimmung des Wasserstoffgehaltes über einen Wärmeleitfähigkeitsdetektor erfolgen. Dies ist in den genannten Konzentrationsbereichen durchaus möglich.

Für den Fall, dass eine Überdosierung des Wasserstoffs stattfindet, schädigt dies die Katalysatoren nachgeordneter Prozesse in der Regel nicht. Dennoch sollten die genannten Obergrenzen an Wasserstoffkonzentration eingehalten werden, da andernfalls in dem Strom enthaltende Wertprodukte durch Hydrierung und/oder Isomerisierung verloren gehen könnten. Die Abtrennung des möglicherweise nicht umgesetzten Wasserstoffs wird bevorzugt in einem bereits vorhandenen destillativen Schritt durchgeführt, z.B. im Kopf der 1-Butendestillation.

Wichtig im Sinne der vorliegenden Erfindung ist, dass das Sorptionsmittel auch in Gegenwart von den genannten Konzentrationen an Wasserstoff keine wesentliche katalytische Aktivität zur Veretherung, Hydrierung, Isomerisierung, Oligomerisierung oder weiteren Reaktionen von Olefinen aufweist. Die genannten Reaktionen der Kohlenwasserstoffe sollen ausschließlich an den dafür vorgesehenen Katalysatoren, nicht aber am Sorptionsmittel erfolgen. Die zu schützenden Katalysatoren befinden sich deswegen vorzugsweise entfernt vom Sorptionsmittel, zumindest in einer anderen Schüttung oder in anderen Apparaten.

Je nach den Umständen werden Kontaktzeiten zwischen 0.01 und 0.2 Stunden vorgesehen, bei Bedarf aber auch darüber hinaus. Da der Betrieb bei erhöhter Temperatur die Abreaktion beschleunigt und die Schwefelkapazität erhöht, ist es vorteilhaft, sie nach den meist vorhandenen Vorwärmern anzuordnen. Die Einhaltung einer bestimmten Temperatur des Sorptionsmittels ist maßgeblich für sein Reinigungsvermögen. Versuche zeigen, dass der Kontakt deswegen bei Temperaturen zwischen 10 °C und 150 °C stattfinden soll, bevorzugt zwischen 20 °C und 130 °C und ganz besonders bevorzugt zwischen 30 °C und 120 °C. Die optimale Kontakttemperatur liegt bei ca. 80 bis 100 °C. Da kommerzielle Methanol-Katalysatoren bei deutlich höheren Temperaturen eingesetzt werden, ist Temperaturstabilität in diesen Bereichen gegeben. Falls der zu schützende Katalysator bei einer anderen Temperatur betrieben wird, sollte das Sorptionsmittel in einem separaten Gefäß, also außerhalb des Reaktors angeordnet werden.

Wichtig ist, dass sich das verunreinigte Kohlenwasserstoffgemisch während des Kontakts mit dem Sorptionsmittel ausschließlich im flüssigen Zustand befindet. Im angegebenen Temperaturbereich wird dies durch einen Druck zwischen 0.5 MPa und 3.5 MPa (entspricht 5 bis 35 bar) gewährleistet. Letztendlich kommt es auf den Druck jedoch nicht an, solange sich die Kohlenwasserstoffe im flüssigen Zustand befinden. Die Raum-/Zeit-Belastung (weight hour space velocity - WHSV) wird dann vorzugsweise zwischen 0.5 h⁻¹ und 20 h⁻¹ gewählt. Dies bedeutet, dass pro Kilogramm Sorptionsmittel zwischen 0.5 und 20 Kilogramm pro Stunde verunreinigtes Kohlenwasserstoffgemisch über das Sorptionsmittel gefahren wird. Das Sorptionsmittel wird in einem Behälter angeschüttet mit einer Schüttdichte im Bereich von 0.7 kg/m³ bis 1.5 kg/m³, bevorzugt etwa 1.15 kg/m³. Durch das Gefäß mit der Schüttung wird das zu reinigende Kohlenwasserstoffgemisch geführt.

Um eine besonders effektive Reinigung zu erreichen und Betriebsunterbrechungen durch Wechsel der Sorptionsmittel zu vermeiden, empfiehlt es sich, mehrere Behälter einzusetzen, die derart revolvierend in Reihe geschaltet werden können, dass am Eintritt immer der Behälter mit der höchsten und zu Austritt hin immer der mit der niedrigsten Beladung angeordnet ist. Mindestens ein Behälter kann dabei, ohne den zu reinigenden Strom zu unterbrechen, herausgenommen und das in ihm befindliche Material gespült und entnommen werden, danach erfolgt in analoger Weise die Neubefüllung.

Das erfindungsgemäße Verfahren eignet sich für die Aufreinigung von Kohlenwasserstoffgemischen, die Olefine mit drei bis acht Kohlenstoffatomen aufweisen. Als technisch relevant sind davon zu erachten z.B. Propen, n-Butene, n-Pentene, Hexene, Neohexen etc sowie deren gesättigte Analoga. Von ihnen nehmen Propan/Propen und die Butane/Butene die absolut bedeutendste Stellung ein. Es wird daher besonders bevorzugt genutzt für die Reinigung von Kohlenwasserstoffgemischen, die Olefine mit drei und/oder vier Kohlenstoffatomen aufweisen. Der Gesamtanteil an Ethen, Olefinen mit mehr als vier Kohlenstoffatomen und Aromaten in dem verunreinigten Kohlenwasserstoffgemisch sollte kleiner sein als 500 Gew-ppm, vorzugsweise kleiner als 50 Gew-ppm.

Das erfindungsgemäße Sorptionsmittel lässt sich besonders vorteilhaft einsetzen zum Reinigen von typischen C₄-Olefinströmen in einem Aufarbeitungszustand unmittelbar vor Umsetzung der darin enthaltenen Butene.

Das Verfahren ist auf solche Gemische besonders gut anwendbar, da es als Gifte für die heterogenen, aluminium-, silicium- oder nickelhaltigen Oligomerisierungskatalysatoren wirkenden, schwefelhaltigen Verunreinigungen gut entfernt.

Bei den Verunreinigungen, die erfindungsgemäß aus dem verunreinigten Kohlenwasserstoffgemisch entfernt werden sollen, handelt es sich vorzugsweise um organische Schwefelverbindungen, die in der nachfolgenden Aufarbeitung des Kohlenwasserstoffgemisches als Katalysatorgift wirken. Neben schwefelhaltigen Verunreinigungen werden auch schwefelfreie Katalysatorgifte wie Basen, Amine oder Nitrile entfernt, wobei diese Stoffe oft unter der Nachweisgrenze liegen.

Schwefelwasserstoff (H₂S), der in rohem Erdgas und Erdöl oft in bedeutsamen Mengen enthalten ist, findet sich in den typischen Chemie-Rohstoffströmen nicht mehr, da er bereits in den Raffinerien bzw. der Erdgas-Aufbereitung abgetrennt wird.

Bei den interessierenden schwefelhaltigen Verunreinigungen, die vorliegend entfernt werden müssen, handelt es sich vielmehr um organische Schwefelverbindungen, die in den Rohstoffströmen der Petro-Folgechemie üblicherweise enthalten sind. Dies sind insbesondere:
a) Thiole mit der allgemeinen Formel R-SH,
b) Disulfide mit der allgemeinen Formel R-S-S-R',
c) Sulfide mit der allgemeinen Formel R-S-R' und
d) substituierte oder unsubstituierte schwefelhaltige Heterozyklen, wie insbesondere Thiophene und/oder Thiolane.

In den oben angegebenen Strukturformeln können R und R' gleiche oder unterschiedliche Alkyl-, Aryl-, Cycloalkyl-, oder Alkenyl-Reste sein, wobei es sich bei R und R' insbesondere um Methyl-, Ethyl-, Propyl-, Butyl-, Phenyl-, Cyclohexyl- oder Butenyl-Reste handelt.

Diese schwefelhaltigen Verunreinigungen werden mit Hilfe der erfindungsgemäßen Reinigung zumindest teilweise, in der Praxis aber sogar vollständig entfernt. Vorzugsweise werden die schwefelhaltigen Verunreinigungen der obigen Substanzklassen zu mehr als 90 Gew.-% entfernt, besonders bevorzugt zu mehr als 95 Gew.-%.

Der besondere Vorteil des erfindungsgemäß eingesetzten Sorptionsmaterials besteht darin, dass es die Verunreinigungen chemisch adsorbiert werden und zwar insbesondere dadurch, dass als Verunreinigung enthaltene Thiole an der Oberfläche des Sorptionsmittels arretiert werden. Die ohne Wasserstoffdosierung auftretende Disulfidbildung aus Thiolen an der Adsorberoberfläche ist formal eine oxidative Kupplung zweier Thiole unter Wasserstoffabspaltung. Durch die hier erfindungsgemäß eingesetzte Wassserstoffdosierung wird das chemische Gleichgewicht deutlich weiter auf die Seite der Thiole verschoben, wodurch die Disulfidbildung deutlich zurückgedrängt wird. Etwaig im C4-Strom bereits vorhandene Disulfide werden somit ebenfalls an dem Sorptionsmittel in ein Thiol umgesetzt und dann arretiert. Die wasserstoffgestützte Chemiesorption bewirkt daher im Vergleich zur Adsorption ohne erfindungsgemäße Wasserstoffdosierung einen besonders hohen Reinigungsgrad, sodass das Kohlenwasserstoffgemisch nahezu vollständig von enthaltenen Thiolen und Disulfiden befreit wird.

Die Chemisorption der Katalysatorgifte ist irreversibel. Aus diesem Grunde kann das erfindungsgemäß eingesetzte Sorptionsmittel nicht regeneriert werden. Dies bedeutet, dass stark verunreinigte Kohlenwasserstoffströme das Sorptionsmittel rasch erschöpfen, sodass es ausgetauscht werden muss. Im Interesse des wirtschaftlichen Betriebs des Reinigungsverfahrens sollte der Gewichtsanteil der Verunreinigungen in dem verunreinigten Kohlenwasserstoffgemisch bezogen auf dessen Gesamtgewicht kleiner als 0.2 Gew.-% sein. Besonders bevorzugt enthält das verunreinigte Kohlenwasserstoffgemisch weniger als 100 Gew.-ppm und besonders bevorzugt weniger als 10 Gew.-ppm Verunreinigungen, jeweils gerechnet als Schwefel. Bei einem solch geringen Verunreinigungsgrad lässt sich das Sorptionsmittel sehr lange betreiben und ermöglicht zudem eine nahezu restlose Entfernung der Katalysatorgifte. Die Maßeinheit ppm versteht sich wie immer als 10⁻⁶.

Nun weisen die üblichen aus Erdölraffinerien stammenden Rohstoffgemische Schwefelgehalte von weit über 0.2 Gew.-% auf. Aus diesem Grunde ist es erforderlich, das Rohstoffgemisch in einer der sorptiven Reinigung vorgeschalteten Vorreinigungsstufe vorzureinigen. In der Vorreinigungsstufe wird das stärker verunreinigte Rohstoffgemisch unter Erhalt eines Kohlenwasserstoffgemisches vorgereinigt, dessen Verunreinigungsgrad unter 0.2 Gew.-% liegt.

Als Vorreinigungsstufe eignet sich insbesondere die oben beschriebene MEROX^{®}-Wäsche oder eine Thioveretherung, wie sie in der WO2014009148A1 offenbart ist.

Die erfindungsmäße Form der Reinigung eignet sich insbesondere dafür, hinter einer MEROX^{®}-Wäsche als Polizei-Filter in den Strom eingeschaltet zu werden.

Unter einem Polizei-Filter ist in diesem Zusammenhang eine zweite Reinigungsinstanz (Feinentschwefelung) zu verstehen, die hinter einer ersten Reinigungsinstanz angeordnet ist und deren Funktion darin besteht, von der ersten Reinigungsinstanz nicht erfasste Restmengen der Katalysatorgifte endgültig von nachfolgenden Reaktionsschritten fernzuhalten oder im Falle einer Betriebsstörung in der ersten Instanz eine sofortige Schädigung der nachfolgenden Reaktionsschritte auszuschließen.

Als erste Reinigungsinstanz dient vorzugsweise eine MEROX^{®}-Wäsche, welche die meisten Katalysatorgifte in größeren Mengen vorweg abscheidet. Lediglich die von der MEROX^{®}-Wäsche nicht erfassten Mercaptane und Disulfide werden dann erfindungsgemäß mit dem hier beschriebenen Sorptionsmittel unter Wasserstoffzugabe zurückgehalten.

Für den Fall einer Betriebsstörung in der Vorreinigung übernimmt der Polizei-Filter die volle Reinigungsfunktion und schützt die Oligomerisierung vor sofortigen irreversiblen Schäden. Da der Polizei-Filter im normalen Betriebszustand nur eine geringe Menge Adsorbat aufnimmt, darf er kapazitiv deutlich kleiner ausgelegt werden als eine typische zur Vorreinigung verwendete MEROX^{®} -Wäsche. Entsprechend rasch ist er bei einem Störfall erschöpft. Die geeignete Dimensionierung des Polizei-Filters hängt davon ab, wie schnell das anströmende Gemisch umgeleitet werden kann.

Thioether als vergleichsweise unreaktive Substanzen werden in MEROX^{®}-Wäschen praktisch nicht entfernt. Um allzu große Konzentrationen beim Kontakt mit dem Adsorptionsmittel zu vermeiden, werden sie vorzugsweise an einer geeigneten Stelle im Prozessablauf vor dem Adsorptionsmittel als Hochsieder in einer Destillation abgetrennt.

In Kombination mit einer Vorreinigungsstufe wie beispielsweise einer MEROX^{®}-Wäsche dann das hier beschriebene Sorptionsmittel bedenkenlos irreversibel eingesetzt werden. Unter einem irreversiblen Einsatz ist in diesem Zusammenhang zu verstehen, dass keine unmittelbare Regeneration, also Wiedergewinnung des aktiven Sorptionsmittels erfolgt, sobald dieses desaktiviert ist. Dies schließt nicht aus, dass das verbrauchte Sorptionsmittel recycelt wird, indem die darin enthaltenen Metalle, wie insbesondere das Kupfer, metallurgisch wiedergewonnen werden. Bei einer solchen metallurgischen Behandlung geht nämlich die ursprüngliche Zusammensetzung des Sorptionsmittels verloren, sodass in diesem Zusammenhang nicht von einer Regeneration gesprochen werden kann.

Das erfindungsgemäße Verfahren eignet sich grundsätzlich zur Entschwefelung von Gemischen aus Kohlenwasserstoffen mit drei bis acht Kohlenstoffatomen. Besonders bevorzugt wird es jedoch zur Entgiftung von C₄-Strömen verwendet, die als Crack C4 oder als FCC C4 oder ihre entsprechenden Raffinate bei der Raffinierung von Erdöl anfallen. Das verunreinigte Kohlenwasserstoffgemisch einen der folgenden, sich jeweils zu 100 Gew.-% ergänzenden Spezifikationen A, B, C oder D, wobei sich die angegebenen Gewichtsanteile jeweils auf das Gesamtgewicht des verunreinigten Kohlenwasserstoffgemisches beziehen.
Spezifikation A:
   - Isobutan 15 bis 45 Gew.-%, bevorzugt 25 bis 35 Gew.-%;
   - n-Butan 5 bis 18 Gew.-%, bevorzugt 8 bis 10 Gew.-%;
   - 1-Buten 5 bis 20 Gew.-%, bevorzugt 12 bis 14 Gew.-%;
   - Isobuten 12 bis 25 Gew.-%, bevorzugt 15 bis 20 Gew.-%;
   - 2-Butene 9 bis 40 Gew.-%, bevorzugt 20 bis 30 Gew.-%;
   - 1.3-Butadien 0 bis 3 Gew.-%, bevorzugt 0.5 bis 0.8 Gew.-%;
   - Wasser 0 bis 1 Gew.-%, bevorzugt weniger als 0.1 Gew.-%;
   - schwefelhaltige Verunreinigungen weniger als 0.5 Gew.-%, bevorzugt unter 0.2 Gew.-%.
   - Wasserstoff weniger als 1 Gew.-ppm.
Spezifikation B:
   - Isobutan 0.5 bis 15 Gew.-%, bevorzugt 1 bis 7 Gew.-%;
   - n-Butan 0.5 bis 20 Gew.-%, bevorzugt 4 bis 7 Gew.-%;
   - 1-Buten 9 bis 25 Gew.-%, bevorzugt 10 bis 20 Gew.-%;
   - Isobuten 10 bis 35 Gew.-%, bevorzugt 20 bis 30 Gew.-%;
   - 2-Butene 3 bis 15 Gew.-%, bevorzugt 5 bis 10 Gew.-%;
   - 1.3-Butadien 25 bis 70 Gew.-%, bevorzugt 40 bis 50 Gew.-%;
   - Wasser 0 bis 1 Gew.-%, bevorzugt weniger als 0.5 Gew.-%;
   - schwefelhaltige Verunreinigungen weniger als 0.5 Gew.-%, bevorzugt unter 0.2 Gew.-%.
   - Wasserstoff weniger als 1 Gew.-ppm.
Spezifikation C:
   - Isobutan 0.5 bis 18 Gew.-%, bevorzugt 1 bis 7 Gew.-%;
   - n-Butan 0.5 bis 25 Gew.-%, bevorzugt 4 bis 13 Gew.-%;
   - 1-Buten 9 bis 40 Gew.-%, bevorzugt 10 bis 35 Gew.-%;
   - Isobuten 10 bis 55 Gew.-%, bevorzugt 20 bis 50 Gew.-%;
   - 2-Butene 3 bis 25 Gew.-%, bevorzugt 5 bis 20 Gew.-%;
   - 1.3-Butadien 0 bis 5 Gew.-%, bevorzugt weniger als 0.8 Gew.-%;
   - Wasser 0 bis 1 Gew.-%, bevorzugt weniger als 0.5 Gew.-%;
   - schwefelhaltige Verunreinigungen weniger als 0.5 Gew.-%, bevorzugt unter 0.2 Gew.-%.
   - Wasserstoff weniger als 1 Gew.-ppm.
Spezifikation D:
   - Isobutan 0 bis 20 Gew.-%, bevorzugt 0 bis 5 Gew.-%;
   - n-Butan 10 bis 35 Gew.-%, bevorzugt 25 bis 30 Gew.-%;
   - 1-Buten 0.2 bis 45 Gew.-%, bevorzugt 3 bis 30 Gew.-%;
   - 2-Butene 35 bis 85 Gew.-%, bevorzugt 50 bis 75 Gew.-%;
   - Wasser 0 bis 1 Gew.-%, bevorzugt weniger als 0.1 Gew.-%;
   - schwefelhaltige Verunreinigungen weniger als 0.5 Gew.-%, bevorzugt unter 0.1 Gew.-%.
   - Wasserstoff weniger als 1 Gew.-ppm.

Die Spezifikation A beschreibt dabei typisches FCC C4, währenddessen die Spezifikation B typisches Crack C4 beschreibt. Spezifikation C beschreibt ein typisches Raffinat I aus Crack C4. Spezifikation D schließlich beschreibt ein Raffinat III aus FCC oder CC4. Da solche technischen C4-Gemische in der Regel frei von Wasserstoff sind, ist der erfindungsgemäß benötigte Wasserstoff vor dem Kontakt hinzuzugeben.

Das erfindungsgemäße Verfahren wird deshalb zur Feinentschwefelung von C4-Gemischen der oben angegebenen Spezifikationen A, B, C oder D genutzt, da das Sorptionsmittel auch in Gegenwart des Wasserstoffs kaum katalytische Aktivität aufweist und damit nicht den wertvollsten Bestandteil, das 1-Buten, umsetzt in Butan oder 2-Buten. Wird das Reinigungsverfahren bei den hier angegebenen Betriebsbedingungen durchgeführt, ist ein Umsatz, d.h. Verlust, an 1-Buten von weniger als 5 % zu erwarten.

Die Erfindung betrifft demnach ein Verfahren, bei dem das verunreinigte Kohlenwasserstoffgemisch 1-Buten enthält, und bei dem durch den Kontakt mit dem Sorptionsmittel weniger als 5 % des im verunreinigten Kohlenwasserstoffgemisch enthaltenden 1-Buten umgesetzt wird.

Nachdem das verunreinigte Kohlenwasserstoffgemisch erfindungsgemäß von seinen Katalysatorgiften befreit wurde, kann die übliche Aufarbeitung solcher Gemische erfolgen, ohne dabei eine Vergiftung der stromabwärts eingesetzten Katalysatoren befürchten zu müssen. Zu den typischen Aufarbeitungsschritten, die auf die hier beschriebene Reinigung folgen können, gehören:
a) Extraktion von im Kohlenwasserstoffgemisch enthaltenden 1.3-Butadien;
b) Selektivhydrierung von im Kohlenwasserstoffgemisch enthaltenden Diolefinen und/oder Acetylenen zu Olefinen;
c) Oligomerisierung von im Kohlenwasserstoffgemisch enthaltenden Olefinen zu entsprechenden Oligomeren;
d) Destillative Abtrennung von im Kohlenwasserstoffgemisch enthaltenden 1-Buten und/oder Isobutan, insbesondere mit dem Zweck, 1-Buten und/oder Isobutan in hoher Reinheit zu erhalten;
e) Entfernung von im Kohlenwasserstoffgemisch enthaltenden Isobuten durch Umwandlung des Isobutens mit Wasser zu tert.-Butanol und/oder mit Methanol zu Methyl-tert.-Butylether;
f) Dehydrierung von im Kohlenwasserstoffgemisch enthaltenden Butanen zu Butenen;
g) oxidative Dehydrierung von im Kohlenwasserstoffgemisch enthaltenden Butenen zu Butadien;
h) Alkylierung von im Kohlenwasserstoffgemisch enthaltenden von n-Buten mit ebenfalls enthaltendem Isobutan;
i) Oxidation von im Kohlenwasserstoffgemisch enthaltenden Kohlenwasserstoffe mit vier Kohlenstoffatomen zur Herstellung von Maleinsäureanhydrid.

Selbstverständlich müssen nicht alle aufgezählten Aufarbeitungsschritte a) bis i) durchgeführt werden, es können auch nur einzelne durchgeführt werden. Auch ist die aufgezählte Reihenfolge nicht bindend.

Darüber hinaus können einzelne der aufgezählten Aufarbeitungsschritte auch vor der erfindungsgemäßen Reinigung angeordnet sein, sofern diese nicht gegen die Katalysatorgifte empfindlich sind. Zumindest eine Nickel-katalysierte Oligomerisierung sollte mit dem erfindungsgemäßen Reinigungsverfahren geschützt werden, da organische Schwefelverbindungen selbst in geringsten Konzentrationen Nickel-Katalysatoren vergiften.

Sofern das eingesetzte Kohlenwasserstoffgemisch auch mit Wasser verunreinigt ist, empfiehlt es sich das mit Wasser verunreinigte Kohlenwasserstoffgemisch vor Kontakt mit dem Sorptionsmittel von Wasser zu befreien, also zu trocknen. Die Abtrennung des Wassers geschieht aus folgender Motivation: Da homogen gelöstes Wasser im Gemisch die Wirkung des Sorptionsmittels etwas abschwächt, wird der Strom vorzugsweise vor dem Kontakt mit dem Sorptionsmittel getrocknet, beispielsweise durch eine Azeotropdestillation (Trocknungsdestillation). Die Trocknung erfolgt tunlichst vor der Zugabe des Wasserstoffes.

### Beispiele

### Erster Versuch: Entfernung von Ethanthiol gemäß der Erfindung

Als Sorptionsmittel wird ein von der Clariant AG erworbener Feststoff eingesetzt, der als Methanol-Katalysator verwendbar ist. Das Sorptionsmittel enthält ca. 42 Gew-% CuO, ca. 44 Gew-% ZnO, ca. 12.5 Gew- % Al₂O₃ und ca. 2 Gew-% Graphit und liegt in Form von Tabletten (5 x 3 mm) vor. Die spezifische Kupferoxidoberfläche beträgt, gemessen mittels Stickstoff-Sorption, 100m² pro g Kupferoxidgehalt.

In ein Reaktionsrohr mit 1 cm Durchmesser werden 27 g Sorptionsmittel gefüllt. Die Schüttdichte beträgt ca. 1.2 kg/dm³. Im Zulauf sowie im Austrag des Rohres ist jeweils ein Probenahmeventil angebracht. Das Sorptionsmittel wird durch Beheizung der Rohrwand auf eine Temperatur von 80 ° C gebracht und bei einem Druck von 21 bar mit einem flüssigen Gemisch durchströmt, das ca. 37 Gew-% 1-Buten, ca. 24 Gew-% trans-2-Buten, ca. 14 Gew-% cis-2-Buten und ca. 24 Gew-% n-Butan sowie 252 Gew-ppm homogen gelösten H₂ enthält. Als Verunreinigung enthält das Material im Mittel 21.8 mg/kg an Schwefel überwiegend in Form von Ethanthiol. Die Belastung des Sorptionsmittels beträgt 357 g/h, der Schwefeleintrag also etwa 7.8 mg/h. Der Schwefel wird ausweislich der Analysen nahezu quantitativ aus dem Gemisch entfernt. Ab einer Betriebszeit von 281 Stunden steigt der Schwefelgehalt am Austrag schnell an. Dieser scharfe Durchbruch entspricht einer arretierten Schwefelmenge von etwa 2.1 g oder einer Schwefelaufnahme des Sorptionsmittels von etwa 7.8 Gew.-%. Die Austragswerte der einzelnen C4-Komponenten blieben gegenüber den entsprechenden Zulaufwerten während der gesamten Versuchszeit nahezu unverändert. Nach dem Ende dieses Versuchs wird die Schüttung mit dem Sorptionsmittel mit Stickstoff gespült. Das Sorptionsmittel kann unversehrt und mit hinreichender Festigkeit entnommen werden. Die Ergebnisse des Versuchs sind in Tabelle 1 niedergelegt.

**Tabelle 1: Ergebnisse aus Versuch 1**

| Mittlerer S-Gehalt [Gew.-%] Zulauf | Mittlerer S-Gehalt [Gew.-%] Austrag bis 281 h | Mittlere Abnahme S [Gew.-%] in Austrag gegenüber Zulauf bis 281 h | mittlerer 1-Buten-Umsatz [%] bis 281 h |
|---|---|---|---|
| 0.00218 | 0.00006 | 97 | 2.3 |

### Zweiter Versuch: Entfernung von Methanthiol gemäß der Erfindung

Das verwendete Sorptionsmittel und der Versuchsaufbau entsprechen dem ersten Versuch. Analog Versuch 1 wird als Verunreinigung im Mittel 20.6 mg/kg an Schwefel überwiegend in Form von Methanthiol zugeführt. Der Gehalt an homogen gelöstem H₂ beträgt 170 Gew-ppm. Die Belastung des Sorptionsmittels, dessen Füllmenge 28 g beträgt, liegt bei 350 g/h, der Schwefeleintrag also etwa 7.2 mg/h. Die Kontakttemperatur wurde auf 80 °C eingestellt. Der Schwefel wird ausweislich der Analysen im Sorptionsmittel nahezu quantitativ aus dem Gemisch entfernt. Ab einer Betriebszeit von etwa 295 Stunden steigt der Schwefelgehalt am Austrag an. Dieser scharfe Durchbruch entspricht einer arretierten Schwefelmenge von etwa 2.1 g oder einer Schwefelaufnahme des Sorptionsmittels von etwa 7.6 Gew.-%. Die Austragswerte der einzelnen C4-Komponenten blieben gegenüber den entsprechenden Zulaufwerten während der gesamten Versuchszeit nahezu unverändert. Nach dem Ende dieses Versuchs werden die Schüttungen mit Stickstoff gespült. Das Sorptionsmittel kann unversehrt und mit hinreichender Festigkeit entnommen werden. Die Versuchsergebnisse sind in Tabelle 2 dargestellt.

**Tabelle 2: Ergebnisse aus Versuch 2**

| Mittlerer S-Gehalt [Gew.-%] Zulauf | Mittlerer S-Gehalt [Gew.-%] Austrag bis 295 h | Mittlere Abnahme S [Gew.-%] in Austrag gegenüber Zulauf bis 295 h | mittlerer 1-Buten-Umsatz [%] bis 295 h |
|---|---|---|---|
| 0.00206 | 0.00007 | 97 | 1.9 |

### Dritter Versuch: Entfernung von Diethyldisulfid gemäß der Erfindung

Das verwendete Sorptionsmittel und der Versuchsaufbau entsprechen dem ersten und zweiten Versuch. Analog Versuch 1 wird als Verunreinigung etwa 1 mg/kg an Schwefel in Form von Diethyldisulfid zugeführt. Die zugeführte Konzentration an homogen gelöstem H₂ beträgt 170 Gew-ppm. Die Belastung der Schüttung, die 27 g des Sorptionsmittels enthält, beträgt 350 g/h, der Schwefeleintrag also etwa 0.35 mg/h. Die Betriebstemperatur beträgt 80 °C. Der Schwefel wird ausweislich der Analysen quantitativ aus dem Gemisch entfernt. Bis zu einer Betriebszeit von 2865 Stunden konnte keinerlei Schwefelkomponente im Austrag detektiert werden. Bis dahin wurden etwa 0.91 g Schwefelmenge arretiert. Dies entspricht einer Schwefelaufnahme des Sorptionsmittels von etwa 3.3 Gew.-% bis zu diesem Zeitpunkt. Die Austragswerte der einzelnen C4-Komponenten blieben gegenüber den entsprechenden Zulaufwerten während der gesamten Versuchszeit nahezu unverändert. Die Versuchsergebnisse sind in Tabelle 3 dargestellt.

**Tabelle 3: Ergebnisse aus Versuch 3**

| Mittlerer S-Gehalt [Gew.-%] Zulauf | Mittlerer S-Gehalt [Gew.-%] Austrag bis 2865 h | Mittlere Abnahme S [Gew.-%] in Austrag gegenüber Zulauf bis 2865 h | mittlerer 1-Buten-Umsatz [%] bis 2865 h |
|---|---|---|---|
| 0.000082 | 0.000000 | 100 | 1.6 |

### Vierter Versuch: Entfernung von Ethanthiol (nicht erfindungsgemäß)

Das verwendete Sorptionsmittel und der Versuchsaufbau entsprechen dem ersten Versuch. Allerdings wird der Versuch ohne Wasserstoffdosierung durchgeführt.

Als Verunreinigung enthält das Material im Mittel 5.4 mg/kg an Schwefel überwiegend in Form von Ethanthiol. Die Belastung der Schüttung, die 120 g des Adsorbers enthält, beträgt 600 g/h, der Schwefeleintrag also etwa 3.2 mg/h.

Der Schwefel wird ausweislich der Analysen zunächst nahezu quantitativ aus dem Gemisch entfernt. Ab einer Betriebszeit von 480 Stunden steigt der Schwefelgehalt am Austrag schnell an. Dieser scharfe Durchbruch entspricht einer arretierten Schwefelmenge von etwa 1.7 g oder einer Schwefelaufnahme des Sorptionsmittels von etwa 1.4 Gew.-%.

Die Austragswerte der einzelnen C₄-Komponenten blieben gegenüber den entsprechenden Zulaufwerten während der gesamten Versuchszeit unverändert.

Nach dem Ende dieses Versuchs wird die Schüttung mit Stickstoff gespült. Das Sorptionsmittel kann unversehrt und mit hinreichender Festigkeit entnommen werden.

Die Ergebnisse des Versuchs sind in Tabelle 4 niedergelegt.

**Tabelle 4: Ergebnisse aus Versuch 4**

| Mittlerer S-Gehalt [Gew.-%] Zulauf | Mittlerer S-Gehalt [Gew.-%] Austrag bis 480 h | Mittlere Abnahme S [Gew.-%] in Austrag gegenüber Zulauf bis 480 h | mittlerer 1-Buten-Umsatz [%] bis 480 h |
|---|---|---|---|
| 0.00054 | 0.00003 | 94 | 0.3 |

### Fazit der Versuche:

Die Versuche belegen, dass das erfindungsgemäß durchgeführte Verfahren durch die Kombination eines geeigneten Sorptionsmittels mit einer Wasserstoffdosierung die folgenden Eigenschaften aufweist:
- Schwefel aus verschiedenen Schwefelverbindungen wird nahezu vollständig gebunden;
- Das Sorptionsmittel benötigt keine Aktivierung im Wasserstoffstrom;
- Das Sorptionsmittel benötigt keine periodischen Reinigungs- und Desorptionsströme, da es sich um ein irreversibles Sorptionsmittel handelt;
- Das Sorptionsmittel kann in einem einfachen Behälter untergebracht werden, der einfach vom dem Gemisch durchströmt wird, vorzugsweise bei leicht erhöhter Temperatur, wie sie typischerweise oft ohnehin für die Speisung von nachfolgenden Reaktoren erforderlich ist;
- Das Verfahren verursacht trotz einer Wasserstoffdosierung praktisch keine Nebenreaktionen der Olefine wie Oligomerisierung, Isomerisierung und Hydrierung und damit auch keine nennenswerten Verluste der Wertbestandteile des zu reinigenden Gemisches;
- Die Wasserstoffdosierung erhöht die Kapazität und damit die Standzeit des Sorptionsmittels gegenüber dem bisher bekannten Stand der Technik;
- Das Verfahren setzt keinerlei Stoffe in Konzentrationen frei, die irgendeinen Einfluss auf die nachfolgenden Verarbeitungsstufen haben;
- Das Verfahren ist angesichts der bei typischen Schwefelkonzentrationen unter 5 Gew.-ppm und der durch die Kapazität des Sorptionsmittels von mindestens 3 Gew.-% Schwefel bedingten langen Lebensdauer sehr kostengünstig im Betrieb, auch wenn das Sorptionsmittel nicht direkt regeneriert werden kann, sondern nach Erschöpfung der Kapazität nur noch einer rohstofflichen Verwertung zugeführt werden kann; diese erscheint aufgrund des hohen Kupfergehalts attraktiv;
- Das Sorptionsmittel lässt sich gefahrlos handhaben und entsorgen, da es weder als kanzerogen eingestuft ist noch pyrophore Eigenschaften zeigt.

## Patentansprüche

1. Verfahren zur Reinigung von Kohlenwasserstoffgemischen, bei welchem ein verunreinigtes Kohlenwasserstoffgemisch enthaltend Olefine mit drei bis acht Kohlenstoffatomen durch Kontaktieren mit einem festen Sorptionsmittel zumindest teilweise von schwefelhaltigen Verunreinigungen befreit wird, wobei sich das Kohlenwasserstoffgemisch während des Kontakts mit dem Sorptionsmittel ausschließlich im flüssigen Zustand befindet, und wobei das Sorptionsmittel die folgende, sich zu 100 Gew.-% ergänzende Zusammensetzung aufweist:
• Kupferoxid: 10 Gew.-% bis 60 Gew.-% (gerechnet als CuO);
• Zinkoxid: 10 Gew.-% bis 60 Gew.-%, (gerechnet als ZnO);
• Aluminiumoxid: 10 Gew.-% bis 30 Gew.-% (gerechnet als Al₂O₃);
• sonstige Stoffe: 0 Gew.-% bis 5 Gew.-%;
**dadurch gekennzeichnet,**
**dass** der Kontakt des verunreinigten Kohlenwasserstoffgemisches mit dem festen Sorptionsmittel in Gegenwart von Wasserstoff erfolgt, wobei das verunreinigte Kohlenwasserstoffgemisch unmittelbar vor dem Kontakt mit dem festen Sorptionsmittel Wasserstoff enthält in einer Konzentration, die auf die Gesamtmasse des verunreinigten Kohlenwasserstoffgemisches bezogen zwischen 1 Gew.-ppm und 10000 Gew.-ppm beträgt, wobei besagte Konzentration an Wasserstoff dadurch eingestellt wird, dass dem verunreinigten Kohlenwasserstoffgemisch unmittelbar vor dem Kontakt Wasserstoff zugegeben wird, wohingegen das verunreinigte Kohlenwasserstoffgemisch vor Zugabe des Wasserstoffs weniger als 1 Gew.-ppm Wasserstoff enthält,
**dass** das verunreinigte Kohlenwasserstoffgemisch 1-Buten enthält, und dass durch den Kontakt mit dem Sorptionsmittel weniger als 5 % des im verunreinigten Kohlenwasserstoffgemisch enthaltenden 1-Buten umgesetzt wird, und
**dass** das verunreinigte Kohlenwasserstoffgemisch eine der folgenden, sich jeweils zu 100 Gew.-% ergänzende Spezifikationen A, B, C oder D erfüllt, wobei sich die angegebenen Gewichtsanteile jeweils auf das Gesamtgewicht des verunreinigten Kohlenwasserstoffgemisches beziehen.
Spezifikation A:
• Isobutan 15 bis 45 Gew.-%, bevorzugt 25 bis 35 Gew.-%;
• n-Butan 5 bis 18 Gew.-%, bevorzugt 8 bis 10 Gew.-%;
• 1-Buten 5 bis 20 Gew.-%, bevorzugt 12 bis 14 Gew.-%;
• Isobuten 12 bis 25 Gew.-%, bevorzugt 15 bis 20 Gew.-%;
• 2-Butene 9 bis 40 Gew.-%, bevorzugt 20 bis 30 Gew.-%;
• 1.3-Butadien 0 bis 3 Gew.-%, bevorzugt 0.5 bis 0.8 Gew.-%;
• Wasser 0 bis 1 Gew.-%, bevorzugt weniger als 0.1 Gew.-%;
• schwefelhaltige Verunreinigungen weniger als 0.5 Gew.-%, bevorzugt unter 0.2 Gew.-%.
• Wasserstoff weniger als 1 Gew.-ppm.
Spezifikation B:
• Isobutan 0.5 bis 15 Gew.-%, bevorzugt 1 bis 7 Gew.-%;
• n-Butan 0.5 bis 20 Gew.-%, bevorzugt 4 bis 7 Gew.-%;
• 1-Buten 9 bis 25 Gew.-%, bevorzugt 10 bis 20 Gew.-%;
• Isobuten 10 bis 35 Gew.-%, bevorzugt 20 bis 30 Gew.-%;
• 2-Butene 3 bis 15 Gew.-%, bevorzugt 5 bis 10 Gew.-%;
• 1.3-Butadien 25 bis 70 Gew.-%, bevorzugt 40 bis 50 Gew.-%;
• Wasser 0 bis 1 Gew.-%, bevorzugt weniger als 0.5 Gew.-%;
• schwefelhaltige Verunreinigungen weniger als 0.5 Gew.-%, bevorzugt unter 0.2 Gew.-%.
• Wasserstoff weniger als 1 Gew.-ppm.
Spezifikation C:
• Isobutan 0.5 bis 18 Gew.-%, bevorzugt 1 bis 7 Gew.-%;
• n-Butan 0.5 bis 25 Gew.-%, bevorzugt 4 bis 13 Gew.-%;
• 1-Buten 9 bis 40 Gew.-%, bevorzugt 10 bis 35 Gew.-%;
• Isobuten 10 bis 55 Gew.-%, bevorzugt 20 bis 50 Gew.-%;
• 2-Butene 3 bis 25 Gew.-%, bevorzugt 5 bis 20 Gew.-%;
• 1.3-Butadien 0 bis 5 Gew.-%, bevorzugt weniger als 0.8 Gew.-%;
• Wasser 0 bis 1 Gew.-%, bevorzugt weniger als 0.5 Gew.-%;
• schwefelhaltige Verunreinigungen weniger als 0.5 Gew.-%, bevorzugt unter 0.2 Gew.-%.
• Wasserstoff weniger als 1 Gew.-ppm.
Spezifikation D:
• Isobutan 0 bis 20 Gew.-%, bevorzugt 0 bis 5 Gew.-%;
• n-Butan 10 bis 35 Gew.-%, bevorzugt 25 bis 30 Gew.-%;
• 1-Buten 0.2 bis 45 Gew.-%, bevorzugt 3 bis 30 Gew.-%;
• 2-Butene 35 bis 85 Gew.-%, bevorzugt 50 bis 75 Gew.-%;
• Wasser 0 bis 1 Gew.-%, bevorzugt weniger als 0.1 Gew.-%;
• schwefelhaltige Verunreinigungen weniger als 0.5 Gew.-%, bevorzugt unter 0.1 Gew.-%.
• Wasserstoff weniger als 1 Gew.-ppm.

2. Verfahren nach Anspruch 1,
**dadurch gekennzeichnet,**
**dass** das verunreinigte Kohlenwasserstoffgemisch unmittelbar vor dem Kontakt mit dem festen Sorptionsmittel Wasserstoff enthält in einer Konzentration, die auf die Gesamtmasse des verunreinigten Kohlenwasserstoffgemisches bezogen zwischen 10 Gew.-ppm und 500 Gew.-ppm beträgt.

3. Verfahren nach Anspruch 2,
**dadurch gekennzeichnet,**
**dass** der Wasserstoff in dem flüssigen verunreinigten Kohlenwasserstoffgemisch vollständig gelöst ist.

4. Verfahren nach Anspruch 1 oder nach einem der Ansprüche 2 bis 3,
**dadurch gekennzeichnet,**
**dass** das Sorptionsmittel die folgende, sich zu 100 Gew.-% ergänzende Zusammensetzung aufweist:
• Kupferoxid: 30 Gew.-% bis 45 Gew.-% (gerechnet als CuO);
• Zinkoxid: 30 Gew.-% bis 50 Gew.-%, (gerechnet als ZnO);
• Aluminiumoxid: 10 Gew.-% bis 15 Gew.-% (gerechnet als Al₂O₃);
• weitere Metalloxide: 0 Gew.-% bis 2 Gew.-%;
• Graphit: 0 Gew.-% bis 3 Gew.-%;
• andere Stoffe: 0 Gew.-% bis 1 Gew.-%.

5. Verfahren nach Anspruch 1 oder nach einem der Ansprüche 2 bis 4,
**dadurch gekennzeichnet,**
**dass** der Kontakt unter den folgenden Bedingungen erfolgt:
• Temperatur zwischen 10 °C und 150 °C, insbesondere zwischen 20 °C und 130 °C und besonders bevorzugt zwischen 30 °C und 120 °C;
• Druck zwischen 0.5 und 3.5 MPa;
• Raum/Zeit-Belastung (weight hour space velocity - WHSV) zwischen 0.5 h⁻¹ und 20 h⁻¹.

6. Verfahren nach Anspruch 1 oder nach einem der Ansprüche 2 bis 5,
**dadurch gekennzeichnet,**
**dass** das verunreinigte Kohlenwasserstoffgemisch als schwefelhaltige Verunreinigung mindestens eine Verbindung der folgenden Substanzklassen enthält:
a) Thiole mit der allgemeinen Formel R-SH
wobei R ein Alkyl-, Aryl-, Cycloalkyl- oder ein Alkenyl-Rest sein kann, wobei es sich bei R insbesondere ein Methyl-, Ethyl-, Propyl-, Butyl-, Phenyl-, Cyclohexyl- oder Butenyl-Rest handelt;
b) Disulfide mit der allgemeinen Formel R-S-S-R',
wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
c) Sulfide mit der allgemeinen Formel R-S-R'
wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
d) Subtitutierte oder unsubstituierte schwefelhaltige Heterocyclen, insbesondere Thiophene und/oder Thiolane.

7. Verfahren nach Anspruch 6,
**dadurch gekennzeichnet,**
**dass** der als Schwefel gerechnete Gewichtsanteil der schwefelhaltigen Verunreinigungen in dem verunreinigten Kohlenwasserstoffgemisch bezogen auf dessen Gesamtgewicht kleiner ist als 0.2 Gew.-%; besonders bevorzugt, dass er unter 100 Gew.-ppm liegt und ganz besonders bevorzugt, dass er unter 10 Gew.-ppm liegt.

8. Verfahren nach Anspruch 7,
**dadurch gekennzeichnet,**
**dass** das verunreinigte Kohlenwasserstoffgemisch durch den Kontakt mit dem Sorptionsmittel von mindestens 90 Gew.-% der im verunreinigten Kohlenwasserstoffgemisch enthaltenden schwefelhaltigen Verunreinigungen befreit wird, vorzugsweise von mindestens 95 Gew.-%.

9. Verfahren nach Anspruch 7 oder 8,
**dadurch gekennzeichnet,**
**dass** das verunreinigte Kohlenwasserstoffgemisch aus einer Vorreinigungsstufe erhalten wird, welche ein stärker verunreinigtes Rohstoffgemisch unter Erhalt des verunreinigten Kohlenwasserstoffgemisches vorreinigt.

10. Verfahren nach Anspruch 9,
**dadurch gekennzeichnet,**
**dass** das Sorptionsmittel irreversibel eingesetzt wird.

11. Verfahren nach Anspruch 1 oder einem der Ansprüche 2 bis 10,
**dadurch gekennzeichnet,**
**dass** das zumindest teilweise von Verunreinigungen befreite Kohlenwasserstoffgemisch zumindest einem der nachfolgend aufgezählten Aufarbeitungsschritte unterworfen wird:
a) Extraktion von im Kohlenwasserstoffgemisch enthaltenden 1.3-Butadien;
b) Selektivhydrierung von im Kohlenwasserstoffgemisch enthaltenden Diolefinen und/oder Acetylenen zu Olefinen;
c) Oligomerisierung von im Kohlenwasserstoffgemisch enthaltenden Olefinen zu entsprechenden Oligomeren;
d) Destillative Abtrennung von im Kohlenwasserstoffgemisch enthaltenden 1-Buten und/oder Isobutan, insbesondere mit dem Zweck, 1-Buten und/oder Isobutan in hoher Reinheit zu erhalten;
e) Entfernung von im Kohlenwasserstoffgemisch enthaltenden Isobuten durch Umwandlung des Isobutens mit Wasser zu tert.-Butanol und/oder mit Methanol zu Methyl-tert.-Butylether;
f) Dehydrierung von im Kohlenwasserstoffgemisch enthaltenden Butanen zu Butenen;
g) oxidative Dehydrierung von im Kohlenwasserstoffgemisch enthaltenden Butenen zu Butadien;
h) Alkylierung von im Kohlenwasserstoffgemisch enthaltenden von n-Buten mit ebenfalls enthaltendem Isobutan;
i) Oxidation von im Kohlenwasserstoffgemisch enthaltenden Kohlenwasserstoffe mit vier Kohlenstoffatomen zur Herstellung von Maleinsäureanhydrid.

## Claims

1. Process for purifying hydrocarbon mixtures, in which a contaminated hydrocarbon mixture comprising olefins having three to eight carbon atoms is at least partly freed of sulfur-containing contaminants by contacting it with a solid sorbent, the hydrocarbon mixture being exclusively in the liquid state during the contact with the sorbent, and wherein the sorbent has the following composition that adds up to 100% by weight:
• copper oxide: 10% by weight to 60% by weight (calculated as CuO);
• zinc oxide: 10% by weight to 60% by weight (calculated as ZnO);
• aluminium oxide: 10% by weight to 30% by weight (calculated as Al₂O₃) ;
• other substances: 0% by weight to 5% by weight;
**characterized in that**
the contaminated hydrocarbon mixture is contacted with the solid sorbent in the presence of hydrogen, where the contaminated hydrocarbon mixture, immediately prior to contact with the solid sorbent, contains hydrogen in a concentration, based on the total mass of the contaminated hydrocarbon mixture, between 1 ppm by weight and 10 000 ppm by weight, where said concentration of hydrogen is established by adding hydrogen to the contaminated hydrocarbon mixture immediately prior to the contact, whereas the contaminated hydrocarbon mixture prior to addition of the hydrogen contains less than 1 ppm by weight of hydrogen,
the contaminated hydrocarbon mixture contains 1-butene, and the contact with the sorbent results in conversion of less than 5% of the 1-butene present in the contaminated hydrocarbon mixture, and
the contaminated hydrocarbon mixture fulfils one of the following specifications A, B, C and D, each of which adds up to 100% by weight, the stated proportions by weight each being based on the total weight of the contaminated hydrocarbon mixture:
Specification A:
• isobutane 15% to 45% by weight, preferably 25% to 35% by weight;
• n-butane 5% to 18% by weight, preferably 8% to 10% by weight;
• 1-butene 5% to 20% by weight, preferably 12% to 14% by weight;
• isobutene 12% to 25% by weight, preferably 150 to 20% by weight;
• 2-butenes 9% to 40% by weight, preferably 20% to 30% by weight;
• 1,3-butadiene 0% to 3% by weight, preferably 0.5% to 0.8% by weight;
• water 0% to 1% by weight, preferably less than 0.1% by weight;
• sulfur-containing impurities less than 0.5% by weight, preferably less than 0.2% by weight;
• hydrogen less than 1 ppm by weight.
Specification B:
• isobutane 0.5% to 15% by weight, preferably 1% to 7% by weight;
• n-butane 0.5% to 20% by weight, preferably 4% to 7% by weight;
• 1-butene 9% to 25% by weight, preferably 10% to 20% by weight;
• isobutene 10% to 35% by weight, preferably 20% to 30% by weight;
• 2-butenes 3% to 15% by weight, preferably 5% to 10% by weight;
• 1,3-butadiene 25% to 70% by weight, preferably 40% to 50% by weight;
• water 0% to 1% by weight, preferably less than 0.5% by weight;
• sulfur-containing impurities less than 0.5% by weight, preferably less than 0.2% by weight;
• hydrogen less than 1 ppm by weight.
Specification C:
• isobutane 0.5% to 18% by weight, preferably 1% to 7% by weight;
• n-butane 0.5% to 25% by weight, preferably 4% to 13% by weight;
• 1-butene 9% to 40% by weight, preferably 10% to 35% by weight;
• isobutene 10% to 55% by weight, preferably 20% to 50% by weight;
• 2-butenes 3% to 25% by weight, preferably 5% to 20% by weight;
• 1,3-butadiene 0% to 5% by weight, preferably less than 0.8% by weight;
• water 0% to 1% by weight, preferably less than 0.5% by weight;
• sulfur-containing impurities less than 0.5% by weight, preferably less than 0.2% by weight;
• hydrogen less than 1 ppm by weight.
Specification D:
• isobutane 0% to 20% by weight, preferably 0% to 5% by weight;
• n-butane 10% to 35% by weight, preferably 25% to 30% by weight;
• 1-butene 0.2% to 45% by weight, preferably 3% to 30% by weight;
• 2-butenes 35% to 85% by weight, preferably 50% to 75% by weight;
• water 0% to 1% by weight, preferably less than 0.1% by weight;
• sulfur-containing impurities less than 0.5% by weight, preferably less than 0.1% by weight;
• hydrogen less than 1 ppm by weight.

2. Process according to Claim 1,
**characterized in that**
the contaminated hydrocarbon mixture, immediately prior to contact with the solid sorbent, contains hydrogen in a concentration, based on the total mass of the contaminated hydrocarbon mixture, between 10 ppm by weight and 500 ppm by weight.

3. Process according to Claim 2,
**characterized in that**
the hydrogen is fully dissolved in the liquid contaminated hydrocarbon mixture.

4. Process according to Claim 1 or either of Claims 2 and 3,
**characterized in that**
the sorbent has the following composition that adds up to 100% by weight:
• copper oxide: 30% by weight to 45% by weight (calculated as CuO);
• zinc oxide: 30% by weight to 50% by weight (calculated as ZnO);
• aluminium oxide: 10% by weight to 15% by weight (calculated as Al₂O₃) ;
• further metal oxides: 0% by weight to 2% by weight;
• graphite: 0% by weight to 3% by weight;
• other substances: 0% by weight to 1% by weight.

5. Process according to Claim 1 or any of Claims 2 to 4,
**characterized in that**
the contact is effected under the following conditions:
• temperature between 10°C and 150°C, especially between 20°C and 130°C and more preferably between 30°C and 120°C;
• pressure between 0.5 and 3.5 MPa;
• space-time yield (weight hourly space velocity - WHSV) between 0.5 h⁻¹ and 20 h⁻¹.

6. Process according to Claim 1 or any of Claims 2 to 5,
**characterized in that**
the contaminated hydrocarbon mixture contains, as sulfur-containing contaminant, at least one compound from one of the following substance classes:
a) thiols having the general formula R-SH where R may be an alkyl, aryl, cycloalkyl or alkenyl radical, where R is especially a methyl, ethyl, propyl, butyl, phenyl, cyclohexyl or butenyl radical;
b) disulfides having the general formula R-S-S-R' where R and R' may be identical or different alkyl, aryl, cycloalkyl or alkenyl radicals, where R and R' are especially methyl, ethyl, propyl, butyl, phenyl, cyclohexyl or butenyl radicals;
c) sulfides having the general formula R-S-R' where R and R' may be identical or different alkyl, aryl, cycloalkyl or alkenyl radicals, where R and R' are especially methyl, ethyl, propyl, butyl, phenyl, cyclohexyl or butenyl radicals;
d) substituted or unsubstituted sulfur-containing heterocycles, especially thiophenes and/or thiolanes.

7. Process according to Claim 6,
**characterized in that**
the proportion by weight of the sulfur-containing contaminants, calculated as sulfur, in the contaminated hydrocarbon mixture, based on the total weight thereof, is less than 0.2% by weight, more preferably below 100 ppm by weight and most preferably below 10 ppm by weight.

8. Process according to Claim 7,
**characterized in that**
the contaminated hydrocarbon mixture is freed by the contact with the sorbent of at least 90% by weight of the sulfur-containing contaminants present in the contaminated hydrocarbon mixture, preferably of at least 95% by weight.

9. Process according to Claim 7 or 8,
**characterized in that**
the contaminated hydrocarbon mixture is obtained from a pre-purification stage which pre-purifies a more highly contaminated raw material mixture to obtain the contaminated hydrocarbon mixture.

10. Process according to Claim 9,
**characterized in that**
the sorbent is used irreversibly.

11. Process according to Claim 1 or any of Claims 2 to 10,
**characterized in that**
the hydrocarbon mixture which has been at least partly freed of contaminants is subjected to at least one of the workup steps enumerated below:
a) extraction of 1,3-butadiene present in the hydrocarbon mixture;
b) selective hydrogenation of diolefins and/or acetylenes present in the hydrocarbon mixture to olefins;
c) oligomerization of olefins present in the hydrocarbon mixture to corresponding oligomers;
d) distillative removal of 1-butene and/or isobutane present in the hydrocarbon mixture, especially with the purpose of obtaining 1-butene and/or isobutane in high purity;
e) removal of isobutene present in the hydrocarbon mixture by conversion of the isobutene with water to tert-butanol and/or with methanol to methyl tert-butyl ether;
f) dehydrogenation of butanes present in the hydrocarbon mixture to butenes;
g) oxidative dehydrogenation of butenes present in the hydrocarbon mixture to butadiene;
h) alkylation of n-butene present in the hydrocarbon mixture with isobutane likewise present;
i) oxidation of hydrocarbons having four carbon atoms present in the hydrocarbon mixture for preparation of maleic anhydride.

## Revendications

1. Procédé de purification de mélanges d'hydrocarbures, dans lequel un mélange d'hydrocarbures contaminé, contenant des oléfines ayant trois à huit atomes de carbone, est au moins partiellement débarrassé des impuretés sulfurées par mise en contact avec un sorbant solide, le mélange d'hydrocarbures se trouvant exclusivement à l'état liquide pendant le contact avec le sorbant, et le sorbant ayant la composition suivante, dont le total fait 100 % en poids :
• oxyde de cuivre : 10 % en poids à 60 % en poids (exprimé en CuO) ;
• oxyde de zinc : 10 % en poids à 60 % en poids (exprimé en ZnO) ;
• oxyde d'aluminium : 10 % en poids à 30 % en poids (exprimé en Al₂O₃) ;
• autres substances : 0 % en poids à 5 % en poids ;
**caractérisé en ce que**
la mise en contact du mélange d'hydrocarbures contaminé avec le sorbant solide s'effectue en présence d'hydrogène, le mélange d'hydrocarbures contaminé contenant, immédiatement avant le contact avec le sorbant, de l'hydrogène à une concentration qui, rapportée à la masse totale du mélange d'hydrocarbures contaminé, est comprise entre 1 ppm en poids et 10 000 ppm en poids, ladite concentration d'hydrogène étant ajustée par addition, avant le contact, d'hydrogène au mélange d'hydrocarbures contaminé, le mélange d'hydrocarbures contaminé contenant en revanche, avant addition de l'hydrogène, moins de 1 ppm en poids d'hydrogène,
**en ce que** le mélange d'hydrocarbures contaminé contient du 1-butène, et **en ce que**, par le contact avec le sorbant, moins de 5 % du 1-butène contenu dans le mélange d'hydrocarbures contaminé sont convertis, et
**en ce que** le mélange d'hydrocarbures contaminé satisfait à l'une des spécifications suivantes A, B, C ou D, dont le total fait 100 % en poids, les proportions en poids indiquées étant chacune rapportées au poids total du mélange d'hydrocarbures contaminé,
Spécification A :
• isobutène 15 à 45 % en poids, de préférence 25 à 35 % en poids ;
• n-butane 5 à 18 % en poids, de préférence 8 à 10 % en poids ;
• 1-butène 5 à 20 % en poids, de préférence 12 à 14 % en poids ;
• isobutène 12 à 25 % en poids, de préférence 15 à 20 % en poids ;
• 2-butènes 9 à 40 % en poids, de préférence 20 à 30 % en poids ;
• 1,3-butadiène 0 à 3 % en poids, de préférence 0,5 à 0,8 % en poids ;
• eau 0 à 1 % en poids, de préférence moins de 0,1 % en poids ;
• impuretés sulfurées moins de 0,5 % en poids, de préférence en dessous de 0,2 % en poids.
• Hydrogène moins de 1 ppm en poids.
Spécification B :
• isobutane 0,5 à 15 % en poids, de préférence 1 à 7 % en poids ;
• n-butane 0,5 à 20 % en poids, de préférence 4 à 7 % en poids ;
• 1-butène 9 à 25 % en poids, de préférence 10 à 20 % en poids ;
• isobutène 10 à 35 % en poids, de préférence 20 à 30 % en poids ;
• 2-butènes 3 à 15 % en poids, de préférence 5 à 10 % en poids ;
• 1,3-butadiène 25 à 70 % en poids, de préférence 40 à 50 % en poids ;
• eau 0 à 1 % en poids, de préférence moins de 0,5 % en poids ;
• impuretés sulfurées moins de 0,5 % en poids, de préférence en dessous de 0,2 % en poids.
• Hydrogène moins de 1 ppm en poids.
Spécification C :
• isobutane 0,5 à 18 % en poids, de préférence 1 à 7 % en poids ;
• n-butane 0,5 à 25 % en poids, de préférence 4 à 13 % en poids ;
• 1-butène 9 à 40 % en poids, de préférence 10 à 35 % en poids ;
• isobutène 10 à 55 % en poids, de préférence 20 à 50 % en poids ;
• 2-butènes 3 à 25 % en poids, de préférence 5 à 20 % en poids ;
• 1,3-butadiène 0 à 5 % en poids, de préférence moins de 0,8 % en poids ;
• eau 0 à 1 % en poids, de préférence moins de 0,5 % en poids ;
• impuretés sulfurées moins de 0,5 % en poids, de préférence en dessous de 0,2 % en poids.
• Hydrogène moins de 1 ppm en poids.
Spécification D :
• isobutane 0 à 20 % en poids, de préférence 0 à 5 % en poids ;
• n-butane 10 à 35 % en poids, de préférence 25 à 30 % en poids ;
• 1-butène 0,2 à 45 % en poids, de préférence 3 à 30 % en poids ;
• 2-butènes 35 à 85 % en poids, de préférence 50 à 75 % en poids ;
• eau 0 à 1 % en poids, de préférence moins de 0,1 % en poids ;
• impuretés sulfurées moins de 0,5 % en poids, de préférence en dessous de 0,1 % en poids.
• Hydrogène moins de 1 ppm en poids.

2. Procédé selon la revendication 1, **caractérisé en ce que** le mélange d'hydrocarbures contaminé contient, immédiatement avant le contact avec le sorbant solide, de l'hydrogène à une concentration qui, rapportée à la masse totale du mélange d'hydrocarbures contaminé, est comprise entre 10 ppm en poids et 500 ppm en poids.

3. Procédé selon la revendication 2, **caractérisé en ce que** l'hydrogène est entièrement dissous dans le mélange d'hydrocarbures contaminé liquide.

4. Procédé selon la revendication 1 ou selon l'une des revendications 2 à 3,
**caractérisé en ce que** le sorbant présente la composition suivante, dont le total fait 100 % en poids :
• oxyde de cuivre : 30 % en poids à 45 % en poids (exprimé en CuO) ;
• oxyde de zinc : 30 % en poids à 50 % en poids (exprimé en ZnO) ;
• oxyde d'aluminium : 10 % en poids à 15 % en poids (exprimé en Al₂O₃) ;
• autres oxydes métalliques : 0 % en poids à 2 % en poids ;
• graphite : 0 % en poids à 3 % en poids ;
• autres substances : 0 % en poids à 1 % en poids.

5. Procédé selon la revendication 1 ou selon l'une des revendications 2 à 4, **caractérisé en ce que** le contact est réalisé dans les conditions suivantes :
• température entre 10 °C et 150 °C, en particulier entre 20 °C et 130 °C et tout particulièrement entre 30 °C et 120 °C ;
• pression entre 0,5 et 3,5 MPa ;
• vitesse spatiale horaire (weight hour space velocity - WHSV) entre 0,5 h⁻¹ et 20 h⁻¹.

6. Procédé selon la revendication 1 ou selon l'une des revendications 2 à 5, **caractérisé en ce que** le mélange d'hydrocarbures contaminé contient, en tant qu'impuretés sulfurées, au moins un composé des classes de substances suivantes :
a) thiols de formule générale R-SH,
dans laquelle R peut représenter un radical alkyle, aryle, cycloalkyle ou alcényle, R représentant en particulier un radical méthyle, éthyle, propyle, butyle, phényle, cyclohexyle ou butényle ;
b) disulfures de formule générale R-S-S-R',
dans laquelle R et R' peuvent représenter des radicaux alkyle, aryle, cycloalkyle ou alcényle identiques ou différents, R et R' représentant en particulier des radicaux méthyle, éthyle, propyle, butyle, phényle, cyclohexyle ou butényle ;
c) sulfures de formule générale R-S-R'
dans laquelle R et R' peuvent représenter des radicaux alkyle, aryle, cycloalkyle ou alcényle identiques ou différents, R et R' représentant en particulier des radicaux méthyle, éthyle, propyle, butyle, phényle, cyclohexyle ou butényle ;
d) composés hétérocycliques sulfurés substitués ou non substitués, en particulier thiophènes et/ou thiolanes.

7. Procédé selon la revendication 6, **caractérisé en ce que** la proportion en poids, exprimée en soufre, des impuretés sulfurées dans le mélange d'hydrocarbures contaminé, rapportée à son poids total, est inférieure à 0,2 % en poids, plus particulièrement **en ce qu'**elle est en dessous de 100 ppm en poids, et tout particulièrement **en ce qu'**elle est en dessous de 10 ppm en poids.

8. Procédé selon la revendication 7, **caractérisé en ce que** le mélange d'hydrocarbures contaminé est, par le contact avec le sorbant, débarrassé d'au moins 90 % en poids des impuretés sulfurées contenues dans le mélange d'hydrocarbures contaminé, de préférence d'au moins 95 % en poids.

9. Procédé selon la revendication 7 ou 8, **caractérisé en ce que** le mélange d'hydrocarbures contaminé est obtenu par une étape de pré-purification, qui assure une pré-purification d'un mélange de matières premières plus fortement contaminé, avec obtention du mélange d'hydrocarbures contaminé.

10. Procédé selon la revendication 9, **caractérisé en ce que** le sorbant est utilisé d'une manière irréversible.

11. Procédé selon la revendication 1 ou l'une des revendications 2 à 10, **caractérisé en ce que** le mélange d'hydrocarbures au moins partiellement débarrassé des impuretés est soumis à au moins l'une des étapes de traitement énumérées ci-après :
a) extraction du 1,3-butadiène contenu dans le mélange d'hydrocarbures ;
b) hydrogénation sélective des dioléfines et/ou des acétylènes contenus dans le mélange d'hydrocarbures, avec obtention d'oléfines ;
c) isomérisation des oléfines contenues dans le mélange d'hydrocarbures, pour obtenir les oligomères correspondants ;
d) séparation par distillation du 1-butène et/ou de l'isobutane contenus dans le mélange d'hydrocarbures, avec en particulier pour objectif d'obtenir du 1-butène et/ou de l'isobutane de haute pureté ;
e) élimination de l'isobutène contenu dans le mélange d'hydrocarbures par conversion de l'isobutène avec de l'eau en tert-butanol et/ou avec du méthanol en méthyl-tert-butyléther ;
f) déshydrogénation des butanes contenus dans le mélange d'hydrocarbures, avec obtention de butènes ;
g) déshydratation oxydative des butènes contenus dans le mélange d'hydrocarbures, avec obtention de butadiène ;
h) alkylation du n-butène contenu dans le mélange d'hydrocarbures, avec l'isobutane qui y est lui aussi contenu ;
i) oxydation des hydrocarbures contenus dans le mélange d'hydrocarbures, à quatre atomes de carbone, pour préparer de l'anhydride maléique.
